# EUROPÄISCHE PATENTSCHRIFT

(11) **EP 2 934 686 B1**
(45) Veröffentlichungstag und Bekanntmachung des Hinweises auf die Patenterteilung: **27.09.2017**
(21) Anmeldenummer: 13792898.2
(22) Anmeldetag: 15.11.2013
(51) Int. Cl.: A61Q 5/10, A61K 8/898, A61K 8/31, A61K 8/25, A61K 8/58, A61K 8/92

(54) **HAARSCHONENDES FÄRBEVERFAHREN MIT SILIKON-VORBEHANDLUNG UND ÖLHALTIGER OXIDATIONSMITTELZUBEREITUNG**
GENTLE HAIR-DYEING METHOD WITH A SILICONE PRE-TREATMENT AND AN OIL-CONTAINING OXIDIZER PREPARATION
PROCÉDÉ DE COLORATION RESPECTUEUX DES CHEVEUX À PRÉTRAITEMENT AU SILICONE ET PRÉPARATION D'AGENT OXYDANT RENFERMANT DE L'HUILE

(30) Priorität: 19.12.2012 DE 102012223809
(43) Veröffentlichungstag der Anmeldung: 28.10.2015
(73) Patentinhaber: Henkel AG & Co. KGaA, 40589 Düsseldorf (DE)
(72) Erfinder: SCHWEINSBERG, Matthias, 22769 Hamburg (DE); BAEK, Jisook, 20144 Hamburg (DE); KLEEN, Astrid, 20457 Hamburg (DE); SCHULZE ZUR WIESCHE, Erik, DE-22453 Hamburg (DE)
(86) Internationale Anmeldenummer: PCT/EP2013/073957
(87) Internationale Veröffentlichungsnummer: WO 2014/095184

(56) Entgegenhaltungen:
- WO-A2-2013/014140
- WO-A2-2013/079299
- US-A- 6 007 801
- US-A- 6 117 420
- US-A1- 2003 121 109
- US-A1- 2008 107 815
- US-B1- 6 315 989
- "Kosmetische Zusammensetzungen", IP.COM JOURNAL, IP.COM INC., WEST HENRIETTA, NY, US, 4. August 2011 (2011-08-04), XP013146799, ISSN: 1533-0001

## Beschreibung

Die vorliegende Erfindung betrifft ein schonendes Verfahren zur oxidativen Haarfärbung, bei dem keratinische Fasern vor oxidativen Einflüssen geschützt werden.

Beim oxidativen Färben von Haaren tritt das Problem auf, dass es aufgrund der aggressiven Agentien zu Irritationen auf der Kopfhaut und Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares; insbesondere die Waschechtheit der Färbung kann durch das Vorbehandlungsmittel verschlechtert sein.

Der vorliegenden Erfindung lag die Aufgabe zugrunde, ein Verfahren zur oxidativen Haarfärbung mit einer Haar schützenden Vorbehandlung bereitzustellen, das die genannten Nachteile überwindet, ohne den Erfolg einer nachfolgenden oxidativen Färbebehandlung zu konterkarieren. Dabei sollten insbesondere ein Verfahren bereitgestellt werden, bei dem das Haar nicht beschwert wird und auch bei einer nicht unmittelbar vor der oxidativen Färbebehandlung stattfindenden Vorbehandlung die gewünschte Wirkung erzielen, wodurch die Zeitspanne zwischen Vorbehandlung und Färben verlängert werden kann.

Der Einsatz von aminierten Silikonen in der Haarpflege ist Stand der Technik. Diese werden in Shampoos und insbesondere in Conditionern breit eingesetzt, um dort Pflegeffekte zu entfalten. So offenbart die EP 1771144 B1 haarkonditionierende Mittel mit aminofunktionellen Siliconen. Die dort beschriebenen Mittel sind Nachbehandlungsmittel.

Auch die europäischen Patente EP 1312334 B1 (Aminosilicon und Verdicker) sowie EP 1312335 B1 (Aminosilicon und Conditioner) offenbaren Haarnachbehandlungsmittel.. Im erstgenannten Dokument werden auch extrem wasserreiche Rezepturen offenbart.

US 2003/121109A1 beschäftigt sich mit der Bereitstellung von oxidativen Haarfärbemitteln, die leuchtende, intensive Färbungen des Haars in einer Vielzahl modischer und natürlicher Nuancen mit guter Beständigkeit gegenüber äußeren Einflüssen, insbesondere einer guten Lichtechtheit und hervorragenden Waschechtheit ermöglichen und die ein sehr gutes Egalisiervermögen und eine verbesserte Grauabdeckung besitzen. Die IP.com-Publikation Nummer IPCOM000209432D vom 04.08.2011 offenbart verschiedene konditioniereride Haarkosmetika, die 4-Morpholinomethyl-substituierte Silikone gemäß der vorliegend beanspruchten Formel (V) enthalten, beschäftigt sich allerdings nicht mit Oxidationsfärbemitteln.

Es wurde nun gefunden, dass eine Vorbehandlung der keratinischen Fasern mit speziellen 4-Morpholinomethyl-substituierten Silikonen innerhalb eines bestimmten Zeitraums vor einer oxidativen Färbebehandlung nicht nur zu deutlich verbessertem Haarschutz führt, ohne dass die Ergebnisse der oxidativen Färbebehandlung beeinträchtigt werden, sondern es werden auch besonders gute Färbeergebnisse, insbesondere Färbungen mit einer hohen Waschechtheit, erzielt. Haarschutz im Sinne der Anmeldung wird insbesondere so verstanden, dass die Struktur der keratinischen Faser, insbesondere des Haars, durch das Oxidationsmittel weniger stark angegriffen wird, so dass die Oberfläche der Faser bzw. des Haars weniger stark aufgeraut wird, die Haarenden weniger stark gesplisst werden, und/oder weniger Haarbruch auftritt.

Gegenstand der vorliegenden Erfindung ist in einer ersten Ausführungsform ein Verfahren zur oxidativen Färbung keratinischer Fasern, insbesondere von Haaren, bei dem
a) ein Vorbehandlungsmittel, das mindestens ein 4-Morpholinomethyl-substituiertes Silikon der Formel (V) enthält, 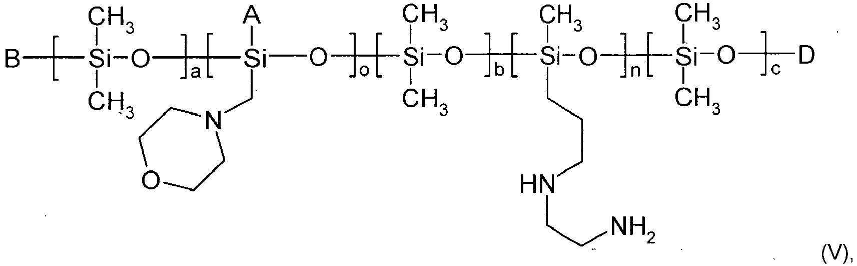 in der
   A für eine über ein -O- gebundene Struktureinheit (I), (II), (IIIa), (IIIb), (IIIe) oder (IIIf) 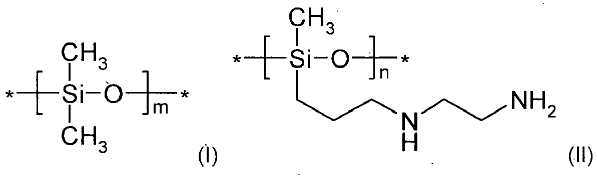 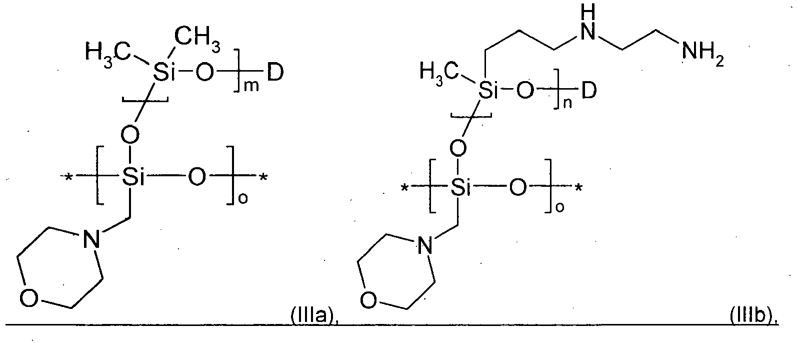 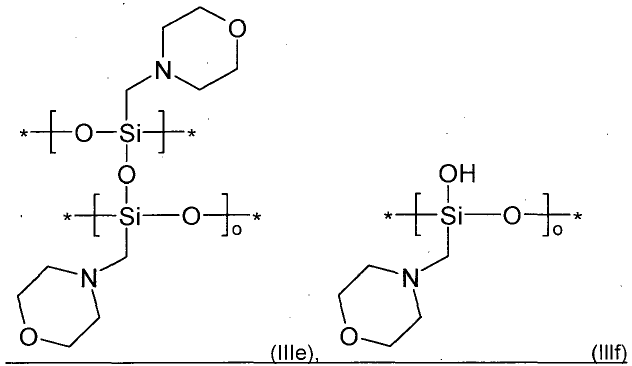 oder für einen über ein -O- gebundenen oligomeren oder polymeren Rest, enthaltend Struktureinheiten der Formeln (I), (II), (IIIa), (IIIb), (IIIe) oder (IIIf) oder für -OH steht,
   * für eine Bindung zu einer der Struktureinheiten (I), (II), (IIIa), (IIIb), (IIIe) oder (IIIf) oder für eine Endgruppe B (Si-gebunden) oder D (O-gebunden) steht,
   B für eine Gruppe -OH, -O-Si(CH₃)₃, -O-Si(CH₃)₂OH, -O-Si(CH₃)₂OCH₃ steht,
   D für eine Gruppe -H, -Si(CH₃)₃, -Si(CH₃)₂OH, -Si(CH₃)₂OCH₃ steht,
   a, b und c für ganze Zahlen von 0 bis 990 stehen, mit der Maßgabe a + b + c > 0 m, n und o für ganze Zahlen von 2 bis 990 stehen, auf die keratinischen Fasern, insbesondere die Haare, aufgetragen wird,
b) anschließend innerhalb eines Zeitraumes von einer Sekunde bis 24 Stunden nach Schritt a) ein Haarfärbemittel auf die keratinischen Fasern aufgebracht wird,
   wobei das Haarfärbemittel durch Vermischen einer Zusammensetzung (A), die mindestens ein Oxidationsfarbstoffvorprodukt vom Entwicklertyp und mindestens ein Oxidationsfarbstoffvorprodukt vom Kupplertyp enthält, mit einer Zusammensetzung (B), die mindestens ein Oxidationsmittel enthält, erhalten wird,
wobei die Zusammensetzung (B) mindestens ein kosmetisches Öl in einer Gesamtmenge von 10-80 Gew.-%, bezogen auf das Gewicht der Zusammensetzung (B), enthält.

Erfindungsgemäß bevorzugt verwendete Vorbehandlungsmittel sind dadurch gekennzeichnet, dass sie 4-Morpholinomethyl-substituierte(s) Silikon(e) in einer Gesamtmenge von 0,001 bis 5 Gew.-%, bevorzugt 0,005 bis 2 Gew.-%, besonders bevorzugt 0,01 - 1 Gew.-%, außerordentlich bevorzugt 0,02 - 0,1 Gew.-%, jeweils bezogen auf das Gesamtgewicht des Vorbehandlungsmittels, enthalten.

Erfindungsgemäß bevorzugt verwendete Vorbehandlungsmittel sind dadurch gekennzeichnet, dass sie das mindestens eine 4-Morpholinomethyl-substituierte Silikon der Formel (V) in in Wasser emulgierter Form enthalten. Besonders bevorzugt verwendete Vorbehandlungsmittel enthalten 30 - 98 Gew.-%, bevorzugt 40 - 90 Gew.-%, besonders bevorzugt 50 - 85 Gew.-%, außerordentlich bevorzugt 60 - 80 Gew.-% Wasser, jeweils bezogen auf das Gesamtgewicht des Vorbehandlungsmittels.

Besonders bevorzugt verwendete Vorbehandlungsmittel liegen in Form einer Öl-in-Wasser-Emulsion vor, in der die zahlenmittlere Größe der Siliconpartikel in der Emulsion im Bereich von 3 bis 500 nm, bevorzugt im Bereich von 5 bis 60 nm liegt.

Die Struktureinheiten der Formeln (I), (II), (IIIa), (IIIb), (IIIe) und (IIIf) können dabei statistisch verteilt im Molekül vorliegen, die erfindungsgemäß eingesetzten Silikone können aber auch Blockcopolymere aus Blöcken der einzelnen Struktureinheiten sein, wobei die Blöcke wiederum statistisch verteilt vorliegen können.

Der * an den freien Valenzen der Struktureinheiten (I), (II), (IIIa), (IIIb), (IIIe) oder (IIIf) steht dabei für eine Bindung zu einer der Struktureinheiten (I), (II), (IIIa), (IIIb), (IIIe) oder (IIIf) oder für eine Endgruppe B (Si-gebunden) oder D (O-gebunden).

Die erfindungsgemäß eingesetzten Silikone können beidseitig Trimethylsilyl-terminiert sein (D = -Si(CH₃)₃, B = -O-Si(CH₃)₃), sie können aber auch ein- oder zweiseitig Dimethylsilylhydroxy- oder Dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h.

sind ausgewählt aus Silikonen, in denen
B = -O-Si(CH₃)₂OH und D = -Si(CH₃)₃
B = -O-Si(CH₃)₂OH und D = -Si(CH₃)₂OH
B = -O-Si(CH₃)₂OH und D = -Si(CH₃)₂OCH₃
B = -O-Si(CH₃)₃ und D = -Si(CH₃)₂OH
B = -O-Si(CH₃)₂OCH₃ und D = -Si(CH₃)₂OH
bedeutet. Diese Silikone führen zu exorbitanten Verbesserungen der Haareigenschaften der nach dem erfindungsgemäßen Verfahren behandelten Haare, insbesondere zu einer gravierenden Verringerung des Kontaktwinkels. 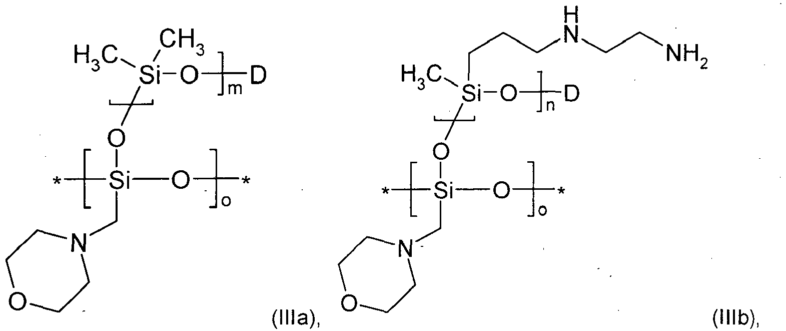 In den vorstehend genannten Formeln (IIIa) und (IIIb) können die Indices m, n und o für ganze Zahlen von 2 bis 990 stehen.

In der Struktureinheit (IIIe) steht A für die Hälfte eines verbindenden O-Atoms zu einer Struktureinheit (III). In der Struktureinheit (IIIf) steht A für -OH.

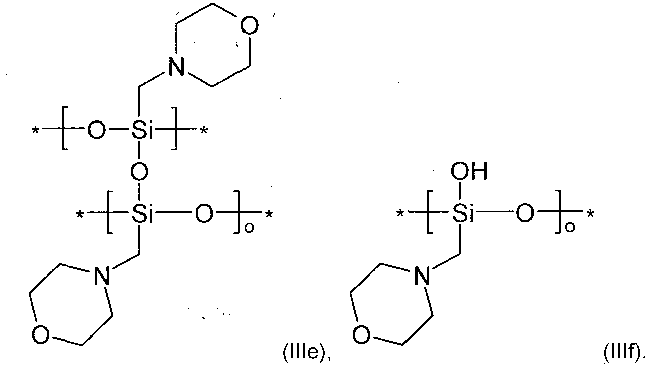

Wie bereits erwähnt, können die Struktureinheiten der Formeln (I), (II), (IIIa), (IIIb), (IIIe) und (IIIf) bevorzugt statistisch verteilt vorliegen.

Strukturformel (V) soll verdeutlichen, dass die Siloxangruppen n und o nicht zwingend direkt an eine Endgruppierung B bzw. D gebunden sein müssen. Vielmehr gilt in bevorzugten Formeln (V) a > 0 oder b > 0 und in besonders bevorzugten Formeln (V) a > 0 und b > 0, d.h. die terminale Gruppierung B bzw. D ist bevorzugt an eine Dimethylsiloxy-Gruppierung gebunden. Auch in Formel (V) sind die Siloxaneinheiten a, b, c, n und o bevorzugt statistisch verteilt.

Auch die durch Formel (V) dargestellten erfindungsgemäß eingesetzten Silikone können beidseitig

Trimethylsilyl-terminiert sein (D = -Si(CH₃)₃, B = -O-Si(CH₃)₃), sie können aber auch ein- oder zweiseitig Dimethylsilylhydroxy- oder Dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen
B = -O-Si(CH₃)₂OH und D = -Si(CH₃)₃
B = -O-Si(CH₃)₂OH und D = -Si(CH₃)₂OH
B = -O-Si(CH₃)₂OH und D = -Si(CH₃)₂OCH₃
B = -O-Si(CH₃)₃ und D = -Si(CH₃)₂OH
B = -O-Si(CH₃)₂OCH₃ und D = -Si(CH₃)₂OH
bedeutet. Diese 4-Morpholinomethyl-substituierte Silikon der Formel (V), das jeweils mindestens eine der Struktureinheiten der Formeln (I), (II) (IIIa), (IIIb), (IIIe) und (IIIf) aufweist, führen zu überraschend hohen Verbesserungen der Haareigenschaften der gemäß dem erfindungsgemäßen Verfahren behandelten Haare, insbesondere zu einem gravierend verbesserten Haarschutz und Farbschutz bei der oxidativen Haarfärbung.

Auch in Formel (V) kann der Rest A
- für eine über ein -O- gebundene Struktureinheit (I), (II) (IIIa), (IIIb), (IIIe) oder (IIIf) oder
- einen über ein -O- gebundenen oligomeren oder polymeren Rest enthaltend Struktureinheiten der Formeln (I), (II) (IIIa), (IIIb), (IIIe) oder (IIIf) stehen.

Analog den Ausführungen zu den Struktureinheiten (IIIa), (IIIb), (IIIe) und (IIIf) wird damit Formel (V) präzisiert zu einer der Formeln (Va), (Vb), (Ve) oder (Vf):

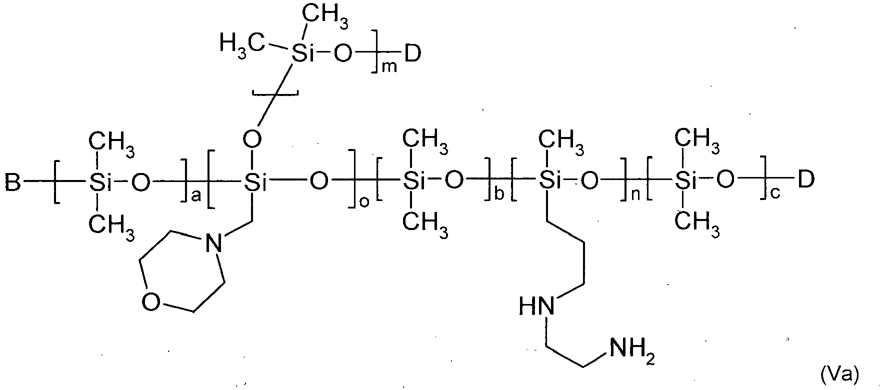

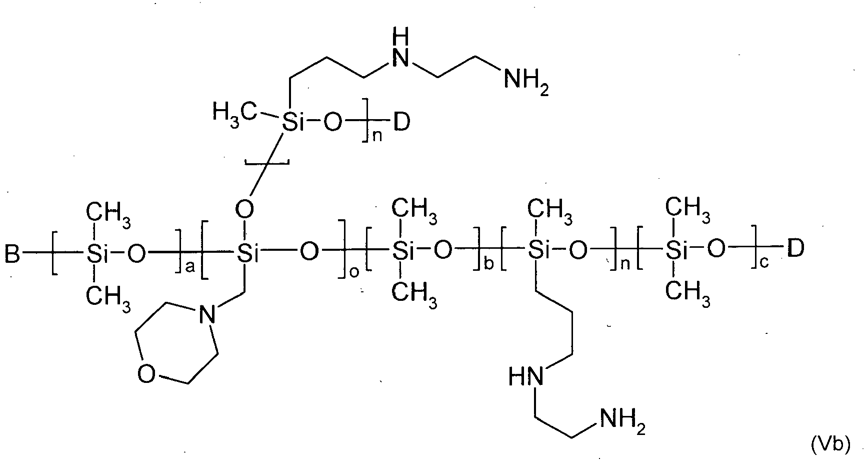

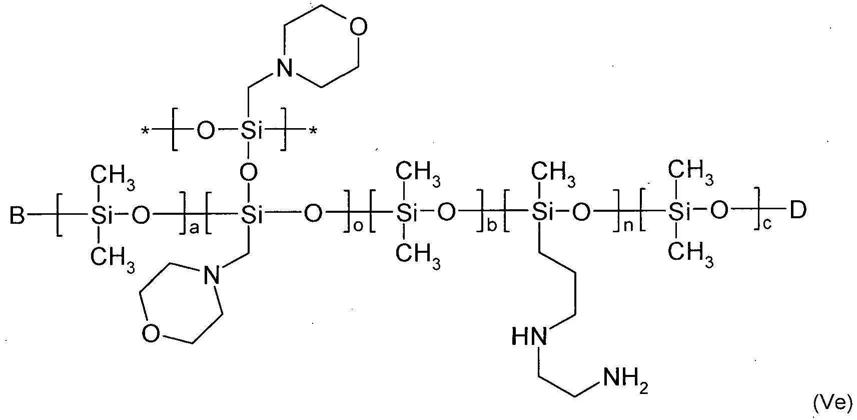

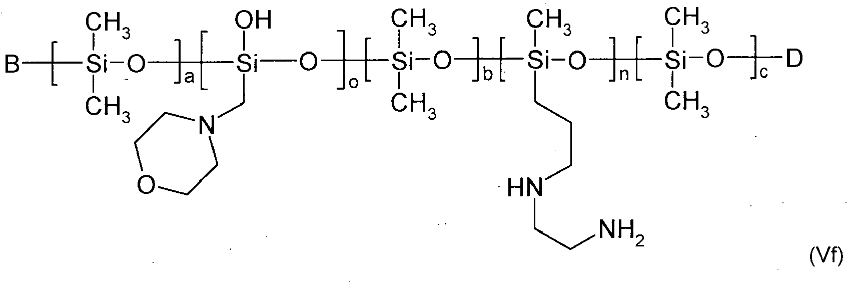

Unabhängig davon, welches spezielle 4-Morpholinomethyl-substituierte Silikon in den erfindungsgemäß verwendeten Vorbehandlungsmitteln enthalten ist, sind für das erfindungsgemäße Verfahren Vorbehandlungsmittel bevorzugt, die ein 4-Morpholinomethyl-substituiertes Silikon enthalten, in dem mehr als 50 Mol-% der Struktureinheiten Dimethylsiloxy-Einheiten sind, d.h. in denen die Struktureinheit (I) mindestens die Hälfte aller Struktureinheiten des eingesetzten Silikons ausmacht.

In anderen Worten sind Silikone bevorzugt, bei denen m > (n + o) bzw. (a+b+c) > (n + o) gilt.

Noch weiter bevorzugte erfindungsgemäß verwendete Vorbehandlungsmittel enthalten ein 4-Morpholinomethyl-substituiertes Silikon, in dem mehr als 87,5 Mol-% der Struktureinheiten Dimethylsiloxy-Einheiten sind, d.h. in denen die Struktureinheit (I) mehr als 875 Tausendstel aller Struktureinheiten des eingesetzten Silikons ausmacht.

In anderen Worten sind Silikone bevorzugt, bei denen m > 8(n + o) bzw. (a+b+c) > 8(n + o) gilt.

Noch weiter bevorzugte erfindungsgemäß verwendete Vorbehandlungsmittel enthalten ein 4-Morpholinomethyl-substituiertes Silikon, in dem mehr als 96 Mol-% der Struktureinheiten Dimethylsiloxy-Einheiten sind, d.h. in denen die Struktureinheit (I) mindestens sechsundneunzig Hundertstel aller Struktureinheiten des eingesetzten Silikons ausmacht.

In anderen Worten sind Silikone bevorzugt, bei denen m > 25(n + o) bzw. (a+b+c) > 25(n + o) gilt.

Noch weiter bevorzugte erfindungsgemäß verwendete Vorbehandlungsmittel enthalten ein 4-Morpholinomethyl-substituiertes Silikon, in dem mehr als 98,7 Mol-% der Struktureinheiten Dimethylsiloxy-Einheiten sind, d.h. in denen die Struktureinheit (I) mindestens neunhundertsiebenundachtzig Tausendstel aller Struktureinheiten des eingesetzten Silikons ausmacht.

In anderen Worten sind Silikone bevorzugt, bei denen m > 77(n + o) bzw. (a+b+c) > 77(n + o) gilt.

Noch weiter bevorzugte erfindungsgemäß verwendete Vorbehandlungsmittel enthalten ein 4-Morpholinomethyl-substituiertes Silikon, in dem mehr als 99,5 Mol-% der Struktureinheiten Dimethylsiloxy-Einheiten sind, d.h. in denen die Struktureinheit (I) mindestens neunhundertfünfundneunzig Tausendstel aller Struktureinheiten des eingesetzten Silikons ausmacht.

In anderen Worten sind Silikone bevorzugt, bei denen m > 200(n + o) bzw. (a+b+c) > 200(n + o) gilt. Zusammenfassend sind bevorzugte erfindungsgemäß verwendete Vorbehandlungsmittel dadurch gekennzeichnet, dass sie mindestens ein 4-Morpholinomethyl-substituiertes Silikon enthalten, bei dem
- m > (n + o) bzw. (a+b+c) > (n + o), bevorzugt
- m > 8(n + o) bzw. (a+b+c) > 8(n + o), besonders bevorzugt
- m > 25(n + o) bzw. (a+b+c) > 25(n + o), weiter bevorzugt
- m > 77(n + o) bzw. (a+b+c) > 77(n + o) und insbesondere
- m > 200(n + o) bzw. (a+b+c) > 200(n + o) gilt.

Ein weiteres erfindungsgemäß bevorzugtes Verfahren ist dadurch gekennzeichnet, dass das in Schritt a eingesetzte Vorbehandlungsmittel Hydroxy-terminierte(s) 4-Morpholinomethyl-substituierte(s) Silikon(e) enthält, in dem/in denen das Molverhältnis Hydroxy/Alkoxy im Bereich von 0,2:1 bis 0,4:1, bevorzugt im Bereich von 1:0,8 bis 1:1,1 liegt.

Ein weiteres erfindungsgemäß bevorzugtes Verfahren ist dadurch gekennzeichnet, dass die gewichtsmittlere Molmasse des in Schritt a eingesetzten 4-Morpholinomethyl-substituierten Silikons der Formel (V) im Bereich von 2.000 bis 1.000.000 gmol⁻¹, bevorzugt im Bereich von 5.000 bis 200.000 gmol⁻¹ liegt.

Die mittleren Molmassen von aminosubstituierten Silikonen sind beispielsweise durch Gelpermeationschromatographie (GPC) bei Raumtemperatur in Polystyrol messbar. Als Säulen können Styragel Spalten µ, als Eluent THF und als Flussrate 1 ml / min gewählt werden. Die Detektion erfolgt bevorzugt mittels Refraktometrie und UV-Meter.

Erfindungsgemäß besonders bevorzugte 4-Morpholinomethyl-substituierte Silikone der Formel (V) sind in dem Rohstoff Belsil ADM 8301 E (ex Wacker Silicones) unter der Bezeichung Amodimethicone/Morpholinomethyl Silsesquioxane enthalten. Belsil ADM 8301 E stellt eine Mikroemulsion dar und besteht aus folgenden Bestandteilen: Amodimethicone/Morpholinomethyl Silsesquioxane (10 Gew.-%); Trideceth-5 (5 Gew.-%); Glycerin (2,5 Gew.-%), Phenoxyethanol (0,45 Gew.-%) und Wasser (82,05 Gew.-%):

Es hat sich gezeigt, dass das erfindungsgemäße Verfahren weiter verbessert werden kann, wenn bestimmte nichtionische Komponenten ebenfalls in den erfindungsgemäß verwendeten Vorbehandlungsmitteln enthalten sind. Zudem haben diese nichtionischen Komponenten positive Effekte auf die Lagerstabilität der erfindungsgemäß verwendeten Vorbehandlungsmittel. Nichtionische Komponenten, die hier besonders geeignet sind, sind Ethoxylate von Decanol, Undecanol, Dodecanol, Tridecanol, Myristylalkohol, Cetylalkohol und/oder Stearylalkohol. Als besonders geeignet haben sich ethoxylierte Tridecanole erwiesen, die mit besonderem Vorzug in die erfindungsgemäß verwendeten Vorbehandlungsmittel inkorporiert werden. Besonders bevorzugt sind verzweigte ethoxylierte Tridecanole, insbesondere verzweigte Tridecanole mit 3 bis 5 Ethylenoxid-Einheiten im Molekül. Erfindungsgemäß besonders bevorzugt verwendete Vorbehandlungsmittel enthalten-jeweils bezogen auf ihr Gewicht - 0,001 bis 5 Gew.-%, bevorzugt 0,005 - 3,5 Gew.-%, besonders bevorzugt 0,01 - 2 Gew.-%, weiter bevorzugt 0,05 - 1 Gew.-% und insbesondere 0,1 - 0,5 Gew.-% verzweigtes, ethoxyliertes Tridecanol, besonders bevorzugt 0,001 - 5 Gew.-%, bevorzugt 0,005-3,5 Gew.-%, besonders bevorzugt 0,01 - 2 Gew.-%, weiter bevorzugt 0,05 -1 Gew.-% und insbesondere 0,1 - 0,5 Gew.-% verzweigtes, ethoxyliertes Tridecanol mit 3 bis 5 Ethylenoxid-Einheiten im Molekül.

Weitere Tenside und Emulgatoren sind in den erfindungsgemäß verwendeten Vorbehandlungsmitteln bevorzugt nicht oder nur in geringen Mengen enthalten. Erfindungsgemäß bevorzugt verwendete Vorbehandlungsmittel enthalten, bezogen auf das Gesamtgewicht des Mittels, 0,001 bis maximal 6 Gew.-% Tensid(e), wobei die vorgenannten Ethoxylate von Decanol, Undecanol, Dodecanol, Tridecanol, Myristylalkohol, Cetylalkohol und/oder Stearylalkohol mit eingeschlossen sind.

Bevorzugt werden die erfindungsgemäß verwendeten Vorbehandlungsmittel niedrigviskos, das heißt, mit einer Viskosität (bei 20°C gemessen) im Bereich von 10 - 2000 mPas, bevorzugt 20-1000 mPas, besonders bevorzugt 50 - 800 mPas, formuliert. Es hat sich darüber hinaus gezeigt, dass verdickende Polymere die erfindungsgemäße Wirkung abschwächen können, so dass bevorzugte erfindungsgemäß verwendete Vorbehandlungsmittel dadurch gekennzeichnet sind, dass sie verdickende Polymere in einer Gesamtmenge von ≤ 2,5 Gew.-%, bevorzugt ≤ 1 Gew.-%, weiter bevorzugt ≤ 0,5 Gew.-% und insbesondere ≤ 0,01 Gew.-%, jeweils bezogen auf das Gewicht des Vorbehandlungsmittels, enthalten.

Die erfindungsgemäß verwendeten Vorbehandlungsmittel können weitere Inhaltsstoffe enthalten. Bevorzugt ist hierbei der Einsatz von mehrwertigen Alkoholen, die Feuchtigkeit spendende Eigenschaften aufweisen. Hier sind erfindungsgemäß verwendete Vorbehandlungsmittel bevorzugt, die mindestens einen mehrwertigen Alkohol, bevorzugt ausgewählt aus der Gruppe Sorbit und/oder Glycerin und/oder 1,2-Propylenglycol oder deren Mischungen, in einer Gesamtmenge von 0,05 - 15 Gew.-%, bevorzugt 0,1 - 10 Gew.-%, besonders bevorzugt 0,15 - 5 Gew.-% und insbesondere 0,15 - 1 Gew.-%, jeweils bezogen auf das Gewicht des Vorbehandlungsmittels, enthalten. Für bestimmte Anwendungsbereiche kann es vorteilhaft sein, nur einen der drei oben genannten bevorzugten mehrwertigen Alkohole einzusetzen. In den meisten Fällen ist dabei Glycerin bevorzugt. Allerdings können auf anderen Anwendungsgebieten Mischungen von zwei der drei mehrwertigen Alkohole oder aller drei mehrwertigen Alkohole bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit, Glycerin und 1,2-Propylenglycol eignen sich als weitere mehrwertige Alkohole solche mit mindestens 2 OH-Gruppen, bevorzugt Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800, eingesetzt werden. Besonders bevorzugt ist der Einsatz von Glycerin, so dass Mittel, die außer Glycerin keine anderen mehrwertigen Alkohole enthalten, besonders bevorzugt sind.

Im Hinblick auf die Vorbehandlung vor einer oxidativen Färbebehandlung ist der Einsatz bestimmter Pflegestoffe in den Vorbehandlungsmitteln des erfindungsgemäßen Verfahrens bevorzugt.

Erfindungsgemäß bevorzugt verwendete Vorbehandlungsmittel sind dadurch gekennzeichnet, dass sie zusätzlich Pflegestoff(e) in einer Gesamtmenge von 0,001 bis 10 Gew.-%, bevorzugt 0,005 bis 7,5 Gew.-%, besonders bevorzugt 0,01 bis 5 Gew.-% und insbesondere 0,05 bis 2,5 Gew.-%, jeweils bezogen auf das Gesamtgewicht des Vorbehandlungsmittels, enthalten. Bevorzugte Pflegestoff(e) sind ausgewählt aus mindestens einer der nachfolgend genannten Gruppen:
i. L-Carnitin und/oder seiner Salze;
ii. Taurin und/oder seiner Salze;
iii. Niacinamid;
iv. Ubichinon;
v. Ectoin;
vi. Vitamine;
vii. Flavonoide.

Als weiteren Inhaltsstoff können die erfindungsgemäß verwendeten Vorbehandlungsmittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3',4'-Dihydroxy-L-phenylalanin (L-Dopa), 5'-Hydroxy-L-tryptophan, L-Homocystein, S-Methyl-L-methionin, *S*-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäß verwendete Vorbehandlungsmittel enthalten eine oder mehrere Aminosäuren in engeren Mengenbereichen. Hier sind erfindungsgemäß verwendete Vorbehandlungsmittel dadurch gekennzeichnet, dass sie als Pflegestoff 0,01 bis 5 Gew.-%, bevorzugt 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), bevorzugt aus der Gruppe Glycin und/oder Alanin und/oder Valin und/oder Lysin und/oder Leucin und/oder Threonin enthalten, jeweils bezogen auf das Gesamtgewicht des Vorbehandlungsmittels.

Das erfindungsgemäß verwendete Vorbehandlungsmittel kann als niedrig-viskose Wasser-basierte Emulsion, als Spray, als Creme, Gel, Lotion, Paste, Shampoo oder Conditioner, konfektioniert sein. Das erfindungsgemäße Verfahren umfasst das Aufbringen eines Vorbehandlungsmittels auf keratinische Fasern und eine sich innerhalb eines Zeitraumes von einer Sekunde bis 24 Stunden daran anschließende oxidative Färbebehandlung.

Ein großer Vorteil der in Schritt a) eingesetzten Vorbehandlungsmittel ist es, dass sie nicht nur dann wirksam sind, wenn sie unmittelbar vor der oxidativen Färbebehandlung angewendet werden, sondern bis zu 24 Stunden vorher angewendet werden können, ohne dass durch äußere Einflüsse eine Abschwächung der Wirkung zu befürchten wäre. Auf diese Weise ist es möglich, beispielsweise morgens nach der Haarwäsche Schritt a) des erfindungsgemäßen Verfahrens durchzuführen und die oxidative Färbebehandlung erst abends.

Erfindungsgemäß bevorzugte Verfahren sind dadurch gekennzeichnet, dass der Zeitraum zwischen den Verfahrensschritten a und b von 2 Sekunden bis 20 Minuten, bevorzugt 30 Sekunden bis 10 Minuten, besonders bevorzugt 1 bis 5 Minuten, beträgt.

Weitere erfindungsgemäß bevorzugte Verfahren sind dadurch gekennzeichnet, dass man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor Verfahrensschritt b erfolgt.

Weitere erfindungsgemäß bevorzugte Verfahren sind dadurch gekennzeichnet, dass man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor mindestens einer der folgenden Verfahrensschritte a)i), die vor dem Verfahrensschritt b) erfolgen, erfolgt:
Ausspülen des Haars;
Trocknen des Haars mit einem Handtuch,
Trocknen lassen des Haars an der Luft;
Trockenfönen des Haars,
Trocknen des Haars mit einer Trockenhaube,
Kombinationen der vorgenannten Verfahrensschritte.

Der Trockenvorgang erfolgt bevorzugt bei einer Temperatur von 20°C bis 150°C.

Dem Trockenvorgang bzw. den Trockenvorgängen geht besonders bevorzugt kein Ausspülen des Haars voraus. Ein erfindungsgemäß bevorzugtes Verfahren ist daher dadurch gekennzeichnet, dass zwischen der in Verfahrensschritt a erfolgten Applikation des Vorbehandlungsmittels und dem Trockenvorgang bzw. den Trockenvorgängen kein Ausspülen des Haars erfolgt. Es kann aber erfindungsgemäß auch bevorzugt sein, nach Schritt a) das Haar erst auszuspülen und anschließend zu trocknen, bevor der Färbeschritt b erfolgt.

Das erfindungsgemäße Verfahren ist weiterhin dadurch gekennzeichnet, dass Schritt b) das Aufbringen eines Haarfärbemittels umfasst, das durch Vermischen einer Zusammensetzung (A), die mindestens ein Oxidationsfarbstoffvorprodukt vom Entwicklertyp und mindestens ein Oxidationsfarbstoffvorprodukt vom Kupplertyp enthält, mit einer Zusammensetzung (B), die mindestens ein Oxidationsmittel enthält, erhalten wird, wobei die Zusammensetzung (B) mindestens ein kosmetisches Öl in einer Gesamtmenge von 10 - 80 Gew.-%, bezogen auf das Gewicht der Zusammensetzung (B), enthält.

Die in dem erfindungsgemäßen Verfahren eingesetzte Zusammensetzung (B) enthält als ersten zwingenden Inhaltsstoff mindestens ein Oxidationsmittel. Bevorzugte Oxidationsmittel sind ausgewählt aus Peroxoverbindungen, bevorzugt ausgewählt aus Wasserstoffperoxid, festen Anlagerungsverbindung von Wasserstoffperoxid an anorganische oder organische Verbindungen, wie Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, weiterhin ausgewählt aus Diammoniumperoxodisulfat (auch als Ammoniumpersulfat bezeichnet), Dinatriumperoxodisulfat (auch als Natriumpersulfat bezeichnet) und Dikaliumperoxodisulfat (auch als Kaliumpersulfat bezeichnet) sowie aus Mischungen dieser Oxidationsmittel. Erfindungsgemäß ganz besonders bevorzugt verwendete Oxidationsmittel sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; bevorzugt werden 6- bis 12-Gewichtsprozentige Lösungen in Wasser verwendet. Erfindungsgemäß bevorzugte Verfahren sind dadurch gekennzeichnet, dass die eingesetzte Zusammensetzung (B) - bezogen auf ihr Gewicht - 1 bis 24 Gew.-%, bevorzugt 4 - 10 Gew.-%, besonders bevorzugt 3 - 6 Gew.-% Wasserstoffperoxid (berechnet als 100%iges H₂O₂) enthält.

Für oxidative Haarfärbeverfahren wird üblicherweise kurz vor der Applikation auf das Haar eine Färbezusammensetzung, die ein oder mehrere Oxidationsfarbstoffvorprodukte und ggf. ein oder mehrere direktziehende Farbstoffe enthält, mit einer wässrigen Oxidationsmittel-haltigen Zusammensetzung zu einem anwendungsbereiten Haarfärbemittel vermischt und dann auf das Haar appliziert. Meistens sind die Färbezusammensetzung und die Oxidationsmittel-haltige Zusammensetzung so aufeinander abgestimmt, dass bei einem Mischungsverhältnis von 1 zu 1, bezogen auf Gewichtsteile, im Haarfärbemittel eine Anfangskonzentration an Wasserstoffperoxid von 0,5 bis 12 Gew.-%, bevorzugt 2 bis 10 Gew.-%, besonders bevorzugt 3 bis 6 Gew.-% Wasserstoffperoxid (berechnet als 100%iges H₂O₂) bezogen auf das Gewicht des Haarfärbemittels vorliegt. Es ist aber genauso gut möglich, die Färbezusammensetzung und die Oxidationsmittel-haltige Zusammensetzung so aufeinander abzustimmen, dass sich die im anwendungsbereiten Färbemittel notwendigen Konzentrationen durch andere Mischungsverhältnisse als 1:1 ergeben, beispielsweise durch ein Gewichts-bezogenes Mischungsverhältnis von 1:2 oder 1:3 oder auch 2:3. Erfindungsgemäß bevorzugte Verfahren sind dadurch gekennzeichnet, dass das in Verfahrensschritt b) eingesetzte anwendungsbereite Haarfärbemittel eine Anfangsmenge an Wasserstoffperoxid von 0,5 - 12 Gew.-%, bevorzugt 2 - 10 Gew.-%, besonders bevorzugt 3 - 6 Gew.-% Wasserstoffperoxid (berechnet als 100%iges H₂O₂) bezogen auf das Gewicht des Haarfärbemittels, enthält.

Weitere erfindungsgemäß bevorzugte Verfahren sind dadurch gekennzeichnet, dass das in Verfahrensschritt b) eingesetzte anwendungsbereite Haarfärbemittel mindestens ein kosmetisches Öl in einer Gesamtmenge von 5 - 50 Gew.-%, bevorzugt 8 - 40 Gew.-% besonders bevorzugt 12 - 30 Gew.-%, außerordentlich bevorzugt 15 - 25 Gew.-%, enthält, jeweils bezogen auf das Gewicht des Haarfärbemittels.

Oxidative Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des in Schritt b) eingesetzten anwendungsbereiten Haarfärbemittels im Bereich von 7 und 11, insbesondere im Bereich von 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten, ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine sind ausgewählt aus der Gruppe 2-Aminoethan-1-ol, 2-Amino-2-methylpropan-l-ol und 2-Amino-2-methyl-propan-1,3-diol.

Für eine Färbung, die eine starke Aufhellung sehr dunklen Haares erfordert, ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Peroxodisulfatsalzen (Persulfatsalzen) eingesetzt. Bevorzugte Persulfatsalze sind Ammoniumperoxidisulfat, Kaliumperoxidisulfat, Natriumperoxidisulfat, sowie Mischungen hiervon. Das mindestens eine Persulfatsalz ist bevorzugt in einer Gesamtmenge von 0,1 bis 25 Gew.-%, besonders bevorzugt in einer Gesamtmenge von 1 bis 15 Gew.-%, bezogen auf das Gewicht des anwendungsbereiten Färbemittels, enthalten.

Als weitere zwingende Komponente enthält die erfindungsgemäß verwendete Zusammensetzung (B) mindestens ein kosmetisches Öl in einer Gesamtmenge von 10 - 80 Gew.-%, bezogen auf das Gewicht der Zusammensetzung (B). Das kosmetische Öl ist unter Normalbedingungen (20 °C, 1013,25 mbar) flüssig; ätherische Öle und Parfümöle bzw. Riechstoffe werden nicht zu den kosmetischen Ölen gezählt. Die unter Normalbedingungen flüssigen kosmetischen Öle sind mit Wasser nicht mischbar. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Sofern in der vorliegenden Anmeldung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist.

Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/ oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Erfindungsgemäß bevorzugte kosmetische Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol^{®}S von BASF).

Weitere erfindungsgemäß bevorzugte kosmetische Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD. Weitere erfindungsgemäß bevorzugte kosmetische Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol^{®} G 16), 2-Octyldodecanol (Eutanol^{®} G), 2-Ethylhexylalkohol und Isostearylalkohol.

Weitere bevorzugte kosmetische Öle sind ausgewählt aus Mischungen aus Guerbetalkoholen und Guerbetalkoholestern, z.B. dem Handelsprodukt Cetiol^{®} PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C₈₋₃₀-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltrüsostearin mit verzweigten Fettsäureresten.

Weitere erfindungsgemäß besonders bevorzugte kosmetische Öle sind ausgewählt aus den Dicarbonsäureestern von linearen oder verzweigten C₂-C₁₀-Alkaholen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol^{®} G 16 S), 2-Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte kosmetische Öle sind ausgewählt aus den Anlagerungsprodukten von 1 bis 5 Propylenoxid-Einheiten an ein- oder mehrwertige C₈₋₂₂-Alkanole, wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Weitere erfindungsgemäß bevorzugte kosmetische Öle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol^{®} CC) oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysilöxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten kosmetischen Öle einzusetzen.

Bevorzugte erfindungsgemäß verwendete Zusammensetzungen (B) sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-lsoparaffinen', sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

Bevorzugte erfindungsgemäße Verfahren sind dadurch gekennzeichnet, dass die erfindungsgemäß verwendete Zusammensetzung (B) mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, enthält, jeweils bezogen auf das Gewicht der Zusammensetzung (B). Weitere bevorzugte erfindungsgemäße Verfahren sind dadurch gekennzeichnet, dass die erfindungsgemäß verwendete Zusammensetzung (B) mindestens ein Tensid enthält.

Bei der Auswahl erfindungsgemäß geeigneter Tenside ist es besonders bevorzugt, ein Gemisch von Tensiden einzusetzen, um die Stabilität der erfindungsgemäß verwendeten Oxidationsmittel-Zusammensetzungen (B) optimal einstellen zu können.

Bevorzugte erfindungsgemäße Verfahren sind dadurch gekennzeichnet, dass das in der Zusammensetzung (B) enthaltene Tensid ausgewählt ist aus nichtionischen Tensiden und anionischen Tensiden sowie aus Mischungen hiervon. Besonders bevorzugt verwendete nichtionische Tenside sind ausgewählt sind aus mit 20 - 100 Mol Ethylenoxid pro Mol ethoxyliertem Rizinusöl, ethoxylierten C₈-C₂₄-Alkanolen mit 10 - 100 Mol Ethylenoxid pro Mol, ethoxylierten C₈-C₂₄-Carbonsäuren mit 10 - 100 Mol Ethylenoxid pro Mol, mit 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen. Bevorzugt ist mit 40 - 80 Mol Ethylenoxid pro Mol ethoxyliertes Rizinusöl in den erfindungsgemäß bevorzugt verwendeten Zusammensetzungen (B) enthalten.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, bevorzugt 10 - 30, besonders bevorzugt 15 bis 25 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12 Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Coceth-30.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, bevorzugt 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Bevorzugte mit 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden bevorzugt C₈ - C₂₂-Alkylmono- und -oligoglycoside eingesetzt. C₈ -C₂₂-Aikyimono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 -2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Als anionische Tenside eignen sich in den erfindungsgemäß verwendeten Zusammensetzungen (B) alle für die Verwendung am menschlichen Körper geeigneten anionischen oberflächenaktiven Stoffe, die eine wasserlöslich machende, anionische Gruppe, beispielsweise eine Carboxylat-, Sulfat-, Sulfonat- oder Phosphat-Gruppe, und eine lipophile Alkylgruppe mit etwa 8 bis 30 C-Atomen, bevorzugt 8 bis 24 C-Atomen im Molekül aufweisen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen), polyethoxylierte Ethercarbonsäuren, Acylsarcoside, Acyltauride, Acylisethionate, Sulfobernsteinsäuremono- und - dialkylester und Sulfobernsteinsäuremono-alkylpolyoxyethylester mit 1 bis 6 Ethylenoxidgruppen, lineare Alkansulfonate, lineare alpha-Olefinsulfonate, Sulfonate ungesättigter Fettsäuren mit bis zu 6 Doppelbindungen, alpha-Sulfofettsäuremethylester von Fettsäuren, C₈-C₂₀-Alkylsulfate und C₈-C₂₀-Alkylethersulfate mit zu bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, sowie Monoglyceridsulfate und Monoglyceridethersulfate.

Bevorzugte anionische Tenside sind Seifen, C₈-C₂₀-Alkylsulfate, C₈-C₂₀-Alkylethersulfate und C₈-C₂₀-Ethercarbonsäuren mit 8 bis 20 C-Atomen in der Alkylgruppe und bis zu 12 Ethylenoxidgruppen im Molekül. Besonders bevorzugt ist Natriumcetearylsulfat.

Bevorzugt beträgt die Gesamtmenge an mindestens einem Tensid in der Oxidationsmittelzusammensetzung (B) 0,1 - 5 Gew.-%, bevorzugt 0,5 - 3 Gew.-% und besonders bevorzugt 1 - 2 Gew.-%, jeweils bezogen auf das Gesamtgewicht der Oxidationsmittelzusammensetzung (B).

Besonders bevorzugt enthält die erfindungsgemäß verwendete Oxidationsmittelzusammensetzung (B) insgesamt 0,1 bis 5 Gew.-%, bevorzugt 0,5 bis 3 Gew.-% und besonders bevorzugt 1 - 2 Gew.-%, jeweils bezogen auf das Gesamtgewicht der Oxidationsmittelzusammensetzung (B), einer Mischung aus nichtionischen und anionischen Tensiden.

Weitere bevorzugte erfindungsgemäße Verfahren sind dadurch gekennzeichnet, dass die erfindungsgemäß verwendete Zusammensetzung (B) mindestens ein lineares gesättigtes Alkanol mit 12 - 30 Kohlenstoffatomen enthält.

Bevorzugte lineare gesättigte Alkanole mit 12 - 30 Kohlenstoffatomen, insbesondere mit 16 - 22 Kohlenstoffatomen, sind ausgewählt aus Cetylalkohol, Stearylalkohol, Arachidylalkohol, Behenylalkohol und Lanolinalkohol sowie Mischungen dieser Alkanole. Erfindungsgemäß besonders bevorzugte Alkanolmischungen sind solche, wie sie bei der technischen Hydrierung von pflanzlichen und tierischen Fettsäuren erhältlich sind. Bevorzugt beträgt die Gesamtmenge an mindestens einem linearen gesättigten Alkanol mit 12 - 30 Kohlenstoffatomen in der Oxidationsmittelzusammensetzung (B) 0,1 - 10 Gew.-%, bevorzugt 0,5 - 7 Gew.-% und besonders bevorzugt 3 - 5 Gew.-%, jeweils bezogen auf das Gesamtgewicht der Oxidationsmittelzusammensetzung (B).

Weitere bevorzugte erfindungsgemäße Verfahren sind dadurch gekennzeichnet, dass die erfindungsgemäß verwendete Zusammensetzung (B):
1 bis 24 Gew.-%, bevorzugt 4 - 10 Gew.-%, besonders bevorzugt 3 - 6 Gew.-% Wasserstoffperoxid (berechnet als 100%iges H₂O₂ weiterhin
mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, weiterhin mindestens ein Tensid in einer Gesamtmenge von 0,1 bis 5 Gew.-%, bevorzugt 0,5 bis 3 Gew.-% und besonders bevorzugt 1 bis 2 Gew.-%, sowie
mindestens ein lineares gesättigtes Alkanol mit 12 - 30 Kohlenstoffatomen in einer Gesamtmenge von 0,1 bis 10 Gew.-%, bevorzugt 0,5 bis 7 Gew.-% und besonders bevorzugt 3 bis 5 Gew.-%, enthält, wobei sich alle Gew.-%-Angaben auf das Gewicht der Zusammensetzung (B) beziehen. Als zwingende Inhaltsstoffe enthält die in dem erfindungsgemäßen Verfahren eingesetzte Zusammensetzung (A) mindestens ein Oxidationsfarbstoffvorprodukt vom Entwicklertyp und mindestens ein Oxidationsfarbstoffvorprodukt vom Kupplertyp.

Oxidationsfarbstoffvorprodukte können aufgrund ihres Reaktionsverhaltens in zwei Kategorien eingeteilt werden, so genannte Entwicklerkomponenten und Kupplerkomponenten.

Kupplerkompohenten bilden im Rahmen der oxidativen Färbung allein keine signifikante Färbung aus, sondern benötigen stets die Gegenwart von Entwicklerkomponenten. Entwicklerkomponenten können mit sich selbst den eigentlichen Farbstoff ausbilden.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride oder Hydrobromide oder der Sulfate einzusetzen.

Überraschend wurde festgestellt, dass mit dem erfindungsgemäßen Verfahren unter Verwendung von mindestens einem Oxidationsfarbstoffvorprodukt vom Entwicklertyp und mindestens einem Oxidationsfarbstoffvorprodukt vom Kupplertyp Haarfärbungen mit besonders hoher Waschechtheit erzielt werden konnten. Eine besonders gute Verbesserung der Waschechtheit wurde für Rezepturen mit der Entwickler/Kuppler-Kombination 1-Hydroxyethyl-4,5-diaminopyrazol/3-Aminophenol beobachtet. Auch die Reduzierung der Haarschädigung war überraschend hoch.

Besonders bevorzugte Entwicklerkomponenten sind ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, den physiologisch verträglichen Salzen dieser Verbindungen sowie Mischungen dieser Entwicklerkomponenten und Entwicklerkomponentensalze.

Ganz besonders bevorzugte Entwicklerkomponenten sind ausgewählt aus 4,5-Diamino-1-(2-hydroxyethyl)pyrazol, p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und Mischungen dieser Verbindungen sowie deren physiologisch verträglichen Salzen. Außerordentlich bevorzugt ist 4,5-Diamino-1-(2-hydroxyethyl)pyrazol und dessen physiologisch verträglichen Salze.

Die Entwicklerkomponenten werden bevorzugt in einer Gesamtmenge von 0,01 -20 Gew.-%, besonders bevorzugt 0,2 - 10 Gew.-%, und außerordentlich bevorzugt 0,6 - 5 Gew.-%, jeweils bezogen auf das Gewicht der Zusammensetzung (A), verwendet.

Die Entwicklerkomponenten werden bevorzugt in einer Gesamtmenge von 0,005 - 10 Gew.-%, besonders bevorzugt 0,1 - 5 Gew.-%, und außerordentlich bevorzugt 0,3 - 2,5 Gew.-%, jeweils bezogen auf das Gewicht des anwendungsbereiten Färbemittels, verwendet.

Unter dem Begriff "anwendungsbereites Färbemittel" wird im Sinne dieser Anmeldung die Mischung aus sämtlichen Oxidationsfarbstoffvorprodukten und sämtlichen Oxidationsmitteln, ggf. in Kombination mit einem geeigneten kosmetischen Träger, z.B. einer Cremebasis, sowie ggf. in Kombination mit mindestens einem direktziehenden Farbstoff, verstanden.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Bevorzugte erfindungsgemäße Verfahren sind dadurch gekennzeichnet, dass das mindestens eine Oxidationsfarbstoffvorprodukt vom Kupplertyp ausgewählt ist aus einer der folgenden Klassen:
- 3-Aminophenol (m-Aminophenol) und/oder dessen Derivate,
- 3-Aminoanilin (m-Diaminobenzol) und/oder dessen Derivate,
- 2-Aminoanilin (1,2-Diaminobenzol; o-Diaminobenzol) und/oder dessen Derivate,
- 2-Aminophenol (o-Aminophenol) und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,

Gemische aus zwei oder mehreren Verbindungen aus einer oder mehreren dieser Klassen sind im Rahmen dieser Ausführungsform ebenso erfindungsgemäß bevorzugt.

Erfindungsgemäß besonders bevorzugte zusätzliche Kupplerkomponenten sind ausgewählt aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol (= 2-Amino-4-Hydroxyethylaminoanisol), 1,3-Bis (2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Ganz besonders bevorzugt sind dabei 3-Aminophenol, Resorcin, 2-Methylresorcin, 5-Amino-2-methylphenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie deren physiologisch verträglichen Salze und Mischungen der genannten Komponenten.

Die mindestens eine Kupplerkomponente wird bevorzugt in einer Gesamtmenge von 0,01 - 20 Gew.-%, besonders bevorzugt 0,2 - 10 Gew.-%, und außerordentlich bevorzugt 0,6 - 5 Gew.-%, jeweils bezogen auf das Gewicht der Zusammensetzung (A), verwendet.

Die mindestens eine Kupplerkomponente wird bevorzugt in einer Gesamtmenge von 0,005 - 10 Gew.-%, bevorzugt 0,1 - 5 Gew.-%, und außerordentlich bevorzugt 0,3 - 2,5 Gew.-%, jeweils bezogen auf das Gewicht des anwendungsbereiten Oxidationsfärbemittels, verwendet.

Im Rahmen der vorliegenden Erfindung sind folgende Kombinationen aus Oxidationsfarbstoffvorprodukten vom Entwicklertyp und vom Kupplertyp besonders bevorzugt, wobei die Aminverbindungen und die Stickstoffheterocyclen auch in Form ihrer physiologisch verträglichen Salze vorliegen können:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanol;
p-Toluylendiamin / 1,3-Bis(2,4-diaminophenoxy)propan;
p-Toluylendiamin / 1-Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethyl-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)ethanol;
N-(4-Amino-3-methylphenyl)-N-[3-(1H-imidazol-1-yl)propyl]amin / 1,3-Bis(2,4-diaminophenoxy)-propan;
N-(4-Amino-3-methylphenyl)-N-[3-(1H-imidazol-1-yl)propyl]amin / 1-Methoxy-2-amino-4-(2-hydroxy-ethylamino)benzol;
N-(4-Amino-3-methylphenyl)-N-[3-(1H-imidazol-1-yl)propyl]amin / 2-Amino-3-hydroxypyridin;
N-(4-Amino-3-methylphenyl)-N-[3-(1H-imidazol-1-yl)propyl]amin / 1-Naphthol; 4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

Erfindungsgemäß besonders bevorzugt sind die Kombinationen 4,5-Diamino-1-(2-hydroxyethyl)-pyrazol/3-Aminophenol und p-Toluylendiamin/3-Aminophenol. Außerordentlich bevorzugt, besonders im Hinblick auf die Verbesserung der Waschechtheit, ist die Kombination 4,5-Diamino-1-(2-hydroxyethyl)pyrazol/3-Aminophenol.

Um eine ausgewogene und subtile Nuancenausbildung zu erzielen, ist es erfindungsgemäß bevorzugt, wenn weitere farbgebende Komponenten in dem Färbemittel enthalten sind, das in dem erfindungsgemäßen Verfahren eingesetzt wird.

In einer weiteren Ausführungsform können die in Schritt b) dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Ein weiteres bevorzugtes erfindungsgemäßes Verfahren ist dadurch gekennzeichnet, dass das in Schritt b applizierte Färbemittel nach einer Zeit von 5-60 Minuten, bevorzugt 30 - 45 Minuten, von den Fasern abgespült wird.

Das im erfindungsgemäßen Verfahren in Schritt b eingesetzte Färbemittel wird aus einem Zweikomponentenmittel hergestellt, wobei eine Komponente, nämlich die Zusammensetzung (A), die Oxidationsfarbstoffvorprodukte und die andere Komponente, nämlich die Zusammensetzung (B), das oder die Oxidationsmittel enthält. Das anwendungsbereite Färbemittel für Schritt b wird dann durch Mischen beider Komponenten direkt vor dem Applikationsschritt b hergestellt. Eine Auftrennung in Mehrkomponentensysteme bietet sich insbesondere dort an, wo Inkompatibilitäten der Inhaltsstoffe zu erwarten oder zu befürchten sind.

Ausführungsbeispiele

### Beispiel 1

Haarsträhnen wurden eine Minute lang in eine wässrige Emulsion eines 4-Morpholinomethyl-substituierten Silikons der Formel (V), die 0,01 Gew.-% 4-Morpholinomethyl-substituierte(s) Silikon(e) der Formel (V) und 0,005 Gew.-% verzweigtes Trideceth-5, weiterhin 0,006 Gew.-% Glycerin und Wasser ad 100 Gew.-% enthielt, eingetaucht und anschließend trocken gefönt.

Anschließend wurde eine frisch zubereitete Färbecreme-Oxidationsmittelmischung auf die Strähnen aufgetragen und 30 Minuten einwirken gelassen. Anschließend wurde das Färbemittel mit Wasser ausgespült. Für die Farbmessungen wurden die Strähnen trocken gefönt. Die Kämmtests wurden an feuchten Strähnen vorgenommen.

**Tabelle 1: Zusammensetzung (A): Färbecreme (Mengenangaben in Gew.-%):**

| | |
|---|---|
| Toluene-2,5-Diamine Sulfate | 0,02 |
| 2-Amino-4-Hydroxyethylaminoanisole Sulfate (1-Methoxy-2-amino-4-(2'-hydroxyethylamino)benzolsulfat) | 0,02 |
| 4-Amino-2-Hydroxytoluene | 0,01 |
| Cetearyl Alcohol | 14 |
| Glyceryl Stearate | 1,4 |
| Ammonium Hydroxide | 6,8 |
| Ceteareth-20 | 3,5 |
| Octyldodecanol | 1 |
| Sodium Laureth Sulfate | 0,5 |
| 1,3-Butylenglycol | 3,5 |
| Sodium Cetearyl Sulfate | 1,0 |
| Oleic Acid | 0,1 |
| Parfum (Fragrance) | 0,5 |
| Potassium Stearate | 0,5 |
| Sodium Sulfite | 0,2 |
| Tetrasodium EDTA | 0,3 |
| Carbomer | 0,3 |
| Polyquaternium-39 (ex Merquat 3330) | 0,05 |
| Potassium Hydroxide | 0,08 |
| Ascorbic Acid | 0,02 |
| Linoleamidopropyl PG-Dimonium Chloride Phosphate | 0,1 |
| Sodium Sulfate | 0,1 |
| Citric Acid | 0,002 |
| CI 77891 (Titanium Dioxide) | 0,3 |
| Aqua (Water, Eau) | ad 100 |

Die vorstehend aufgeführte Zusammensetzung (A) = Färbecreme 12-0 wurde im Gewichtsverhältnis 1:1 entweder mit der nachstehend dargestellten Zusammensetzung (B)-E = Oxidationsmittelzusammensetzung (B) mit erfindungsgemäßem Ölgehalt oder mit der nachstehend dargestellten Zusammensetzung (B)-V = Oxidationsmittelzusammensetzung (B) mit geringerem Ölgehalt jeweils zu einem anwendungsfertigen Färbemittel vermischt. Anschließend wurde das anwendungsbereite Färbemittel auf die Teststrähnen appliziert, und zwar je 4 g Färbemittel pro Gramm Haar. Dabei wurden zum einen Teststrähnen verwendet, die zuvor mit dem 4-Morpholinomethyl-substituierten Silikon der Formel (V) behandelt worden waren, zum anderen wurden unbehandelte Teststrähnen eingesetzt.

Das Färbemittel verblieb jeweils 30 Minuten auf den Strähnen. Anschließend wurden die Strähnen mit 32°C warmem Leitungswasser mit einer Fließgeschwindigkeit von 0,5 Liter pro Minute 2 Minuten lang ausgespült.

**Tabelle 2: Für das erfindungsgemäße Verfahren verwendete Zusammensetzung (B)-E (Mengenangaben in Gew.-%):**

| | |
|---|---|
| Paraffinum liquidum | 17,00 |
| Cetearyl Alcohol | 4,00 |
| Dipicolinsäure | 0,10 |
| Disodium Pyrophosphate | 0,10 |
| Potassium Hydroxide | 0,12 |
| Etidronic Acid | 0,20 |
| PEG-40 Castor Oil | 0,70 |
| Sodium Cetearyl Sulfate | 0,40 |
| H₂O₂ (Aktivgehalt) | 6,00 |
| Wasser | ad 100,00 |

**Tabelle 3: Für das Vergleichsverfahren verwendete Zusammensetzung (B)-V (Mengen in Gew.-%):**

| | |
|---|---|
| Paraffinum Liquidum | 0,5 |
| Cetearylalkohol | 4,0 |
| Dipicolinsäure | 0,1 |
| Disodium Pyrophosphate | 0,1 |
| Kaliumhydroxid | 0,1 |
| 1,2-Propylenglycol | 1,0 |
| 1-Hydroxyethane-1,1-diphosphonic acid (Etidronic acid) | 0,1 |
| STEARTRIMONIUM CHLORIDE | 0,5 |
| Ceteareth-20 | 1,0 |
| H₂O₂ (Aktivgehalt) | 12,0 |
| Wasser | ad 100 |

**Tabelle 4: Nasskämmbarkeit; Kämmarbeit in mJ**

| | Kämmarbeit [mJ] | Relative Änderung [%] |
|---|---|---|
| (A) + (B)-V, ohne Verfahrensschritt a (Vergleich) | 1248 | 100 |
| (A) + (B)-E, ohne Verfahrensschritt a (Vergleich) | 905 | 73 |
| (A) + (B)-V, mit Verfahrensschritt a (Vergleich) | 976 | 78 |
| (A) + (B)-E, mit Verfahrensschritt a (erfindungsgemäß) | 728 | 58 |

**Tabelle 5: Splissanzahl nach 20000 Kämmungen (Anteil in %)**

| | Splissanzahl [%] | Relative Änderung [%] |
|---|---|---|
| (A) + (B)-V, ohne Verfahrensschritt a (Vergleich) | 1,8 | 100 |
| (A) + (B)-E, ohne Verfahrensschritt a (Vergleich) | 1,3 | 72 |
| (A) + (B)-V, mit Verfahrensschritt a (Vergleich) | 1,1 | 61 |
| (A) + (B)-E, mit Verfahrensschritt a (erfindungsgemäß) | 0,9 | 50 |

Wie die in den Tabellen 4 und 5 dargestellten Daten zeigen, wirken sich sowohl die Vorbehandlung mit einem 4-Morpholinomethyl-substituierten Silikon der Formel (V) allein als auch die Verwendung einer Oxidationsmittelzusammensetzung (B) mit hohem Ölgehalt allein bereits positiv auf den Schutz des Haares vor oxidativer Schädigung aus: sowohl die Kämmarbeit wird um 20 bzw. 27 % reduziert, wobei eine geringere Kämmarbeit gleichbedeutend ist mit einer geringeren Haarschädigung; als auch die durch ein standardisiertes Testkämmen verursachte Splisszahl wird um 28 bzw. 39 % reduziert.

Demgegenüber bringt das erfindungsgemäße Verfahren, das die Vorbehandlung mit dem 4-Morpholinomethyl-substituierten Silikon der Formel (V) mit der Verwendung einer Oxidationsmittelzusammensetzung (B) mit hohem Ölgehalt kombiniert, eine weitere sehr deutlich Reduktion der Nasskämmarbeit und der Splissrate.

## Patentansprüche

1. Verfahren zur oxidativen Färbung keratinischer Fasern, insbesondere von Haaren, **dadurch gekennzeichnet, dass**
a) ein Vorbehandlungsmittel, das mindestens ein 4-Morpholinomethyl-substituiertes Silikon der Formel (V) enthält, 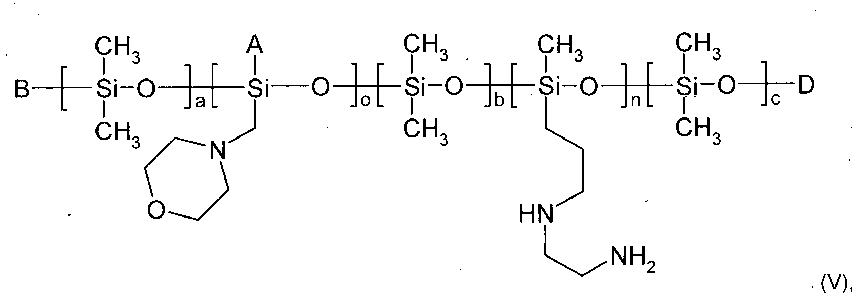 in der
A für eine über ein -O- gebundene Struktureinheit (I), (II), (IIIa), (IIIb), (IIIe) oder (IIIf), 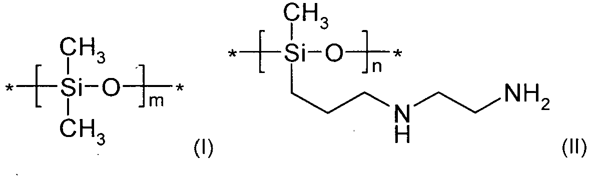 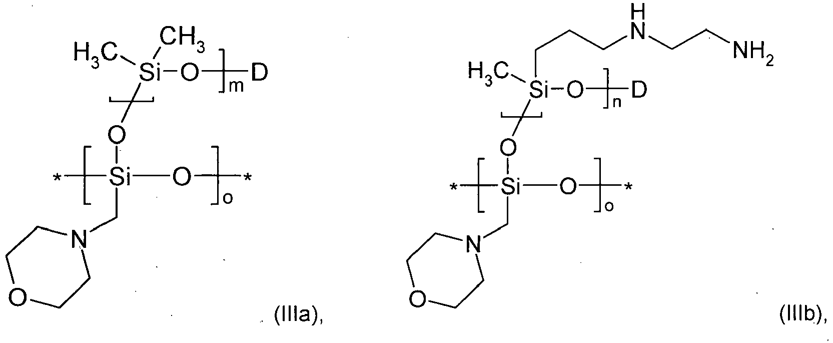 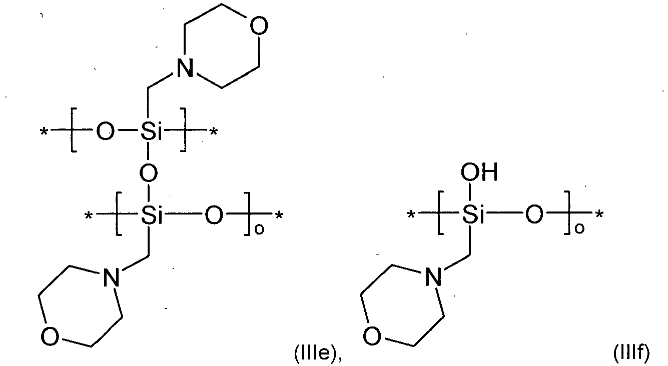 oder für einen über ein -O-gebundenen oligomeren oder polymeren Rest, enthaltend Struktureinheiten der Formeln (I), (II), (IIIa), (IIIb), (IIIe) oder (IIIf) oder für -OH steht,
* für eine Bindung zu einer der Struktureinheiten (I), (II), (IIIa), (IIIb), (IIIe) oder (IIIf) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H, -Si(CH₃)₃, -Si(CH₃)₂OH, -Si(CH₃)₂OCH₃ steht,
a, b und c für ganze Zahlen von 0 bis 990 stehen, mit der Maßgabe a + b + c > 0
m, n und o für ganze Zahlen von 1 bis 990 stehen,
auf die keratinischen Fasern, insbesondere die Haare, aufgetragen wird,
b) anschließend innerhalb eines Zeitraumes von einer Sekunde bis 24 Stunden nach Schritt a) ein Haarfärbemittel auf die keratinischen Fasern aufgebracht wird,
wobei das Haarfärbemittel durch Vermischen einer Zusammensetzung (A), die mindestens ein Oxidationsfarbstoffvorprodukt vom Entwicklertyp und mindestens ein Oxidationsfarbstoffvorprodukt vom Kupplertyp enthält, mit einer Zusammensetzung (B), die mindestens ein Oxidationsmittel enthält, erhalten wird,
**dadurch gekennzeichnet, dass** die Zusammensetzung (B) mindestens ein kosmetisches Öl in einer Gesamtmenge von 10 - 80 Gew.-%, bezogen auf das Gewicht der Zusammensetzung (B), enthält.

2. Verfahren nach Anspruch 1, **dadurch gekennzeichnet, dass** das in Schritt a eingesetzte Vorbehandlungsmittel mindestens ein 4-Morpholinomethyl-substituiertes Silikon der Formel (V) enthält, bei dem
m > (n + o) bzw. (a+b+c) > (n + o), bevorzugt
m > 8(n + o) bzw. (a+b+c) > 8(n + o), besonders bevorzugt
m > 25(n + o) bzw. (a+b+c) > 25(n + o), weiter bevorzugt
m > 77(n + o) bzw. (a+b+c) > 77(n + o) und insbesondere
m > 200(n + o) bzw. (a+b+c) > 200(n + o) gilt.

3. Verfahren nach einem der Ansprüche 1 oder 2, **dadurch gekennzeichnet, dass** das in Schritt a eingesetzte Vorbehandlungsmittel, bezogen auf sein Gewicht, 4-Morpholinomethyl-substituierte(s) Silikon(e) in einer Gesamtmenge von 0,001 bis 5 Gew.-%, bevorzugt 0,005 bis 2 Gew.-%, besonders bevorzugt 0,01 -1 Gew.-%, außerordentlich bevorzugt 0,02 - 0,1 Gew.-%, jeweils bezogen auf das Gesamtgewicht des Vorbehandlungsmittels, enthält.

4. Verfahren nach einem der Ansprüche 1 bis 3, **dadurch gekennzeichnet, dass** das in Schritt a eingesetzte Vorbehandlungsmittel Hydroxy-terminierte(s) 4-Morpholinomethyl-substituierte(s) Silikon(e) enthält, in denen das Molverhältnis Hydroxy/Alkoxy im Bereich von 0,2:1 bis 0,4:1, bevorzugt im Bereich von 1:0,8 bis 1:1,1 liegt.

5. Verfahren nach einem der Ansprüche 1 bis 4, **dadurch gekennzeichnet, dass** die gewichtsmittlere Molmasse des in Schritt a eingesetzten 4-Morpholinomethyl-substituierten Silikons der Formel (V) im Bereich von 2.000 bis 1.000.000 gmol⁻¹, bevorzugt im Bereich von 5.000 bis 200.000 gmol⁻¹ liegt.

6. Verfahren nach einem der Ansprüche 1 bis 5, **dadurch gekennzeichnet, dass** das in Schritt a eingesetzte 4-Morpholinomethyl-substituierte Silikon der Formel (V) in Form einer Öl-in-Wasser-Emulsion vorliegt, in der die zahlenmittlere Größe der Siliconpartikel in der Emulsion bevorzugt im Bereich von 3 bis 500 nm, besonders bevorzugt im Bereich von 5 bis 60 nm liegt.

7. Verfahren nach einem der Ansprüche 1 bis 6, **dadurch gekennzeichnet, dass** das mindestens eine kosmetische Öl, das in der Zusammensetzung (B) in einer Gesamtmenge von 10 - 80 Gew.-%, bezogen auf das Gewicht der Zusammensetzung (B), enthalten ist, ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isopar-affinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

8. Verfahren nach einem der Ansprüche 1 bis 7, **dadurch gekennzeichnet, dass** die Zusammensetzung (B) mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, enthält, jeweils bezogen auf das Gewicht der Zusammensetzung (B).

9. Verfahren nach einem der Ansprüche 1 bis 8, **dadurch gekennzeichnet, dass** die Zusammensetzung (B):
1 bis 24 Gew.-%, bevorzugt 4 - 10 Gew.-%, besonders bevorzugt 3 - 6 Gew.-% Wasserstoffperoxid (berechnet als 100%iges H₂O₂), weiterhin
mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, weiterhin
mindestens ein Tensid in einer Gesamtmenge von 0,1 - 5 Gew.-%, bevorzugt 0,5 - 3 Gew.-% und besonders bevorzugt 1 - 2 Gew.-%, sowie
mindestens ein lineares gesättigtes Alkanol mit 12 - 30 Kohlenstoffatomen in einer Gesamtmenge von 0,1 - 10 Gew.-%, bevorzugt 0,5 - 7 Gew.-% und besonders bevorzugt 3 - 5 Gew.-%, enthält, wobei sich alle Gew.-%-Angaben auf das Gewicht der Zusammensetzung (B) beziehen.

10. Verfahren nach einem der Ansprüche 1 bis 9, **dadurch gekennzeichnet, dass** das in Verfahrensschritt b) eingesetzte anwendungsbereite Haarfärbemittel mindestens ein kosmetisches Öl in einer Gesamtmenge von 5 - 50 Gew.-%, bevorzugt 8 - 40 Gew.-% besonders bevorzugt 12 - 30 Gew.-%, außerordentlich bevorzugt 15 - 25 Gew.-%, enthält, jeweils bezogen auf das Gewicht des Haarfärbemittels.

11. Verfahren nach einem der Ansprüche 1 bis 10, **dadurch gekennzeichnet, dass** man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor mindestens einer der folgenden Verfahrensschritte a)i), die vor dem Verfahrensschritt b) erfolgen, erfolgt:
Ausspülen des Haars;
Trocknen des Haars mit einem Handtuch,
Trocknen lassen des Haars an der Luft;
Trockenfönen des Haars,
Trocknen des Haars mit einer Trockenhaube,
Kombinationen der vorgenannten Verfahrensschritte,
wobei der Trockenvorgang bevorzugt bei einer Temperatur von 20°C bis 150°C erfolgt.

12. Verfahren nach Anspruch 11, **dadurch gekennzeichnet, dass** zwischen der in Verfahrensschritt a erfolgten Applikation des Vorbehandlungsmittels und dem Trockenvorgang bzw. den Trockenvorgängen kein Ausspülen des Haars erfolgt.

13. Verfahren nach einem der Ansprüche 1 bis 12, **dadurch gekennzeichnet, dass** der Zeitraum zwischen den Verfahrensschritten a und b 30 Sekunden bis 20 Minuten, bevorzugt 45 Sekunden bis 10 Minuten, besonders bevorzugt 1 bis 5 Minuten, beträgt.

14. Verfahren nach einem der Ansprüche 1 bis 13, **dadurch gekennzeichnet, dass** die in Schritt b aufgetragene Kombination aus mindestens einem Oxidationsfarbstoffvorprodukt vom Entwicklertyp und mindestens einem Oxidationsfarbstoffvorprodukt vom Kuppertyp ausgewählt ist aus mindestens einer der folgenden Kombinationen, wobei die Aminverbindungen und die Stickstoffheterocyclen auch in Form ihrer physiologisch verträglichen Salze vorliegen können:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanoi;
p-Toluylendiamin / 1,3-Bis(2,4-diaminophenoxy)propan;
p-Toluylendiamin / 1-Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethy.l-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)-ethanol;
N-(4-Amino-3-methylphenyl)-N-[3-(1H-imidazol-1-yl)propyl]amin / 1,3-Bis(2,4-diaminophenoxy)-propan;
N-(4-Amino-3-methylphenyl)-N-[3-(1H-imidazol-1-yl)propyl]amin / 1-Methoxy-2-amino-4-(2-hydroxyethylamino)benzol;
N-(4-Amino-3-methylphenyl)-N-[3-(1H-imidazol-1-yl)propyl]amin / 2-Amino-3-hydroxypyridin;
N-(4-Amino-3-methylphenyl)-N-[3-(1H-imidazol-1-yl)propyl]amin / 1-Naphthol;
4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

## Claims

1. A method for oxidatively dyeing keratin fibers, in particular hair, **characterized in that**
a) a pretreatment agent which contains at least one 4-morpholinomethyl-substituted silicone of formula (V), 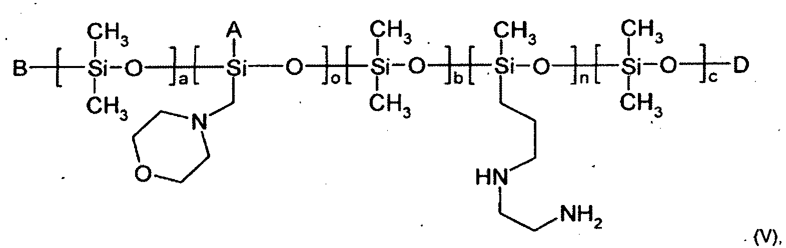 in which
A represents a structural unit (I), (II), (IIIa), (IIIb), (IIIe) or (IIIf) bonded by an -O-, 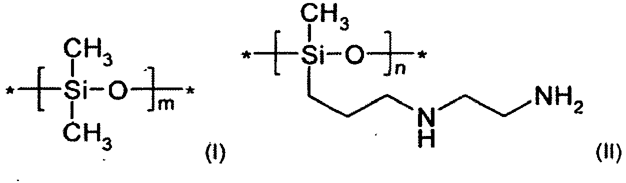 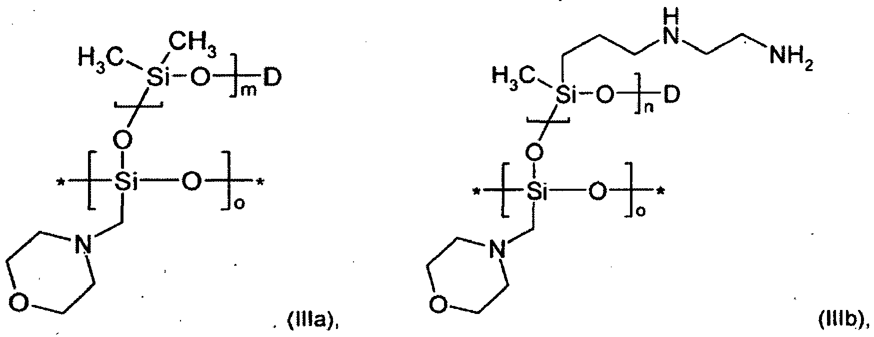 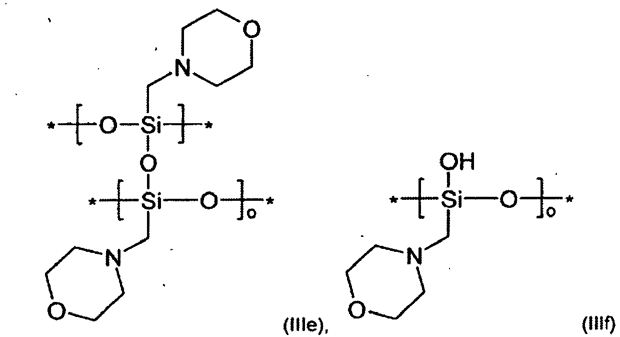 or an oligomeric or polymeric functional group bonded by an -O-, containing structural units of formulas (I), (II), (IIIa), (IIIb), (IIIe) or (IIIf), or represents -OH,
* a bond to one of the structural units (I), (II), (IIIa), (IIIb), (IIIe) or (IIIf) or an end group B (Si-bonded) or D (O-bonded),
B represents a group -OH, -O-Si(CH₃)₃, -O-Si(CH₃)₂OH, -O-Si(CH₃)₂OCH₃,
D represents a group -H, -Si(CH₃)₃, -Si(CH₃)₂OH, -Si(CH₃)₂OCH₃,
a, b and c represent integers of from 0 to 990, with the proviso a + b + c > 0
m, n and o represent integers of between 1 and 990,
is applied to the keratin fibers, in particular the hair,
b) subsequently, within a period of from one second to 24 hours, after step a),
a hair dye is applied to the keratin fibers,
the hair dye being obtained by mixing a composition (A), which contains at least one developer-type oxidation dye precursor and at least one coupler-type oxidation-dye precursor, with a composition (B), which contains at least one oxidizing agent,
**characterized in that** the composition (B) contains at least one cosmetic oil in a total amount of from 10-80 wt.% based on the weight of the composition (B).

2. The method according to claim 1, **characterized in that** the pretreatment agent used in step a contains at least one 4-morpholinomethyl-substituted silicone of formula (V), in which m > (n + o) or (a+b+c) > (n+o), preferably
m> 8(n+o) or (a+b+c) > 8(n+o), particularly preferably
m > 25(n + o) or (a+b+c) > 25(n+o), more preferably
m > 77(n+o) or (a+b+c) > 77(n+o) and in particular
m > 200(n+o) or (a+b+c) > 200(n+o).

3. The method according to one of claims 1 or 2, **characterized in that** the pretreatment agent used in step a contains, based on its weight, 4-morpholinomethyl-substituted silicone(s) in a total amount of from 0.001 to 5 wt.%, preferably 0.005 to 2 wt.%, particularly preferably 0.01-1 wt.%, most particularly preferably 0.02-0.1 wt.%, each based on the total weight of the pretreatment agent.

4. The method according to one of claims 1 to 3, **characterized in that** the pretreatment agent used in step a contains hydroxy-terminated 4-morpholinomethyl-substituted silicone(s), in which the hydroxy/alkoxy molar ratio is in the range of from 0.2:1 to 0.4:1, preferably in the range of from 1:0.8 to 1:1.1.

5. The method according to one of claims 1 to 4, **characterized in that** the weight-average molecular weight of the 4-morpholinomethyl-substituted silicone of formula (V) used in step a is in the range of from 2,000 to 1,000,000 gmol⁻¹, preferably in the range of from 5,000 to 200,000 gmol-1

6. The method according to one of claims 1 to 5, **characterized in that** the 4-morpholinomethyl-substituted silicone of formula (V) used in step a is in the form of an oil-in-water emulsion, in which the number-average size of the silicone particles in the emulsion is preferably in the range of from 3 to 500 nm, particularly preferably in the range of from 5 to 60 nm.

7. The method according to one of claims 1 to 6, **characterized in that** the at least one cosmetic oil, contained in the composition (B) in a total amount of from 10-80 wt.% based on the weight of the composition (B), is selected from natural and synthetic hydrocarbons, particularly preferably from paraffin oils, C₁₈-C₃₀ isoparaffins, in particular isoeicosane, polyisobutene and polydecene, C₈-C₁₆ isoparaffins, as well as 1,3-di-(2-ethylhexyl)-cyclohexane; benzoic acid esters of linear or branched C₈₋₂₂ alkanols; fatty alcohols having 6-30 carbon atoms which are unsaturated or branched and saturated or branched and unsaturated; triglycerides of linear or branched, saturated or unsaturated, optionally hydroxylated C₈₋₃₀ fatty acids, in particular natural oils; dicarboxylic acid esters of linear or branched C₂₋C₁₀ alkanols; esters of linear or branched saturated or unsaturated fatty alcohols having 2 to 30 carbon atoms with linear or branched, saturated or unsaturated fatty acids having 2 to 30 carbon atoms, which may be hydroxylated; addition products of 1 to 5 propylene oxide units to mono- or polyvalent C₈₋₂₂ alkanols; addition products of at least 6 ethylene oxide and/or propylene oxide units to mono- or polyvalent C₃₋₂₂ alkanols; C₈-C₂₂ fatty alcohol esters of monovalent or polyvalent C₂-C₇ hydroxycarboxylic acids; symmetrical, asymmetrical or cyclic esters of carbonic acid having C₃₋₂₂ alkanols, C₃₋₂₂ alkanediols or C₃₋₂₂ alkanetriols; esters of dimers of unsaturated C₁₂-C₂₂ fatty acids (dimer fatty acids) having monovalent linear, branched or cyclic C₂-C₁₈ alkanols or having polyvalent linear or branched C₂-C₆ alkanols; silicone oils as well as mixtures of the aforementioned substances.

8. The method according to one of claims 1 to 7, **characterized in that** the composition (B) contains at least one cosmetic oil in a total amount of from 12-70 wt.%, preferably 14-60 wt.%, particularly preferably 15-52 wt.% and most particularly preferably 17-35 wt.%, based on the weight of the composition (B) in each case.

9. The method according to one of claims 1 to 8, **characterized in that** the composition (B):
contains 1 to 24 wt.%, preferably 4-10 wt.%, particularly preferably 3-6 wt.% of hydrogen peroxide (calculated as 100% H₂O₂), furthermore
at least one cosmetic oil in a total amount of from 12-70 wt.%, preferably 14-60 wt.%, particularly preferably 15-52 wt.% and most particularly preferably 17-35 wt.%, furthermore
at least one surfactant in a total amount of from 0.1-5 wt.%, preferably 0.5-3 wt.% and particularly preferably 1-2 wt.%, as well as
at least one linear, saturated alkanol having 12-30 carbon atoms in a total amount of from 0.1-10 wt.%, preferably 0.5-7 wt.% and particularly preferably 3-5 wt.%, where all wt.% values refer to the weight of the composition (B).

10. The method according to one of claims 1 to 9, **characterized in that** the ready-to-apply hair dye used in method step b) contains at least one cosmetic oil in a total amount of from 5-50 wt.%, preferably 8-40 wt.%, particularly preferably 12-30 wt.%, most particularly preferably 15-25 wt.%, based on the weight of the hair dye in each case.

11. The method according to one of claims 1 to 10, **characterized in that** the pretreatment agent applied in method step a is left on the hair for a period of from 2 seconds to 120 minutes, preferably 5 seconds to 10 minutes, before at least one of the following method steps a)i), which are carried out before method step b), takes place:
rinsing out the hair;
drying the hair with a towel,
leaving the hair to air-dry;
blow drying the hair,
drying the hair with a drying hood,
combinations of the aforementioned method steps,
the drying process preferably being carried out at a temperature of from 20 °C to 150 °C.

12. The method according to claim 11, **characterized in that** the hair is not rinsed out between the application of the pretreatment agent in method step a and the drying process or drying processes.

13. The method according to one of claims 1 to 12, **characterized in that** the time period between the method steps a and b is 30 seconds to 20 minutes, preferably 45 seconds to 10 minutes, particularly preferably 1 to 5 minutes.

14. The method according to one of claims 1 to 13, **characterized in that** the combination, applied in step b, of at least one developer-type oxidation dye precursor and at least one coupler-type oxidation dye precursor is selected from at least one of the following combinations, in which the amine compounds and the nitrogen heterocycles can be present in the form of their physiologically acceptable salts:
p-toluylenediamine / resorcinol;
p-toluylenediamine / 2-methylresorcinol;
p-toluylenediamine / 5-amino-2-methylphenol;
p-toluylenediamine / 3-aminophenol;
p-toluylenediamine / 2-(2,4-diaminophenoxy)ethanol;
p-toluylenediamine / 1,3-bis(2,4-diaminophenoxy)propane;
p-toluylenediamine / 1-methoxy-2-amino-4-(2-hydroxyethylamino)benzene;
p-toluylenediamine / 2-amino-3-hydroxypyridine;
p-toluylenediamine / 1-naphthol;
2-(2-hydroxyethyl)-p-phenylenediamine / resorcinol
2-(2-hydroxyethyl)-p-phenylenediamine / 2-methylresorcinol;
2-(2-hydroxyethyl)-p-phenylenediamine / 5-amino-2-methylphenol;
2-(2-hydroxyethyl)-p-phenylenediamine / 3-aminophenol;
2-(2-hydroxyethyl)-p-phenylenediamine / 2-(2,4-diaminophenoxy)ethanol;
2-(2-hydroxyethyl)-p-phenylenediamine / 1,3-bis(2,4-diaminophenoxy)propane;
2-(2-hydroxyethyl)-p-phenylenediamine / 1-methoxy-2-amino-4-(2-hydroxyethylamino)benzene;
2-(2-hydroxyethyl)-p-phenylenediamine / 2-amino-3-hydroxypyridine;
2-(2-hydroxyethyl)-p-phenylenediamine / 1-naphthol;
2-methoxymethyl-p-phenylenediamine / resorcinol;
2-methoxymethyl-p-phenylenediamine / 2-methylresorcinol;
2-methoxymethyl-p-phenylenediamine / 5-amino-2-methylphenol;
2-methoxymethyl-p-phenylenediamine / 3-aminophenol;
2-methoxymethyl-p-phenylenediamine / 2-(2,4-diaminophenoxy)ethanol;
2-methoxymethyl-p-phenylenediamine / 1,3-bis(2,4-diaminophenoxy)propane;
2-methoxymethyl-p-phenylenediamine / 1-methoxy-2-amino-4-(2-hydroxyethylamino)benzene;
2-methoxymethyl-p-phenylenediamine / 2-amino-3-hydroxypyridine;
2-methoxymethyl-p-phenylenediamine / 1-naphthol;
N-(4-amino-3-methylphenyl)-N-[3-(1H-imidazol-1-yl)propyl]amine / resorcinol;
N-(4-amino-3-methylphenyl)-N-[3-(1H-imidazol-1-yl)propyl]amine / 2-methylresorcinol;
N-(4-amino-3-methylphenyl)-N-[3-(1H-imidazol-1-yl)propyl]amine / 5-amino-2-methylphenol;
N-(4-amino-3-methylphenyl)-N-[3-(1H-imidazol-1-yl)propyl]amine / 3-aminophenol;
N-(4-amino-3-methylphenyl)-N-[3-(1H-imidazol-1-yl)propyl]amine / 2-(2,4-diaminophenoxy)-ethanol;
N-(4-amino-3-methylphenyl)-N-[3-(1H-imidazol-1-yl)propyl]amine / 1,3-bis(2,4-diaminophenoxy)-propane;
N-(4-amino-3-methylphenyl)-N-[3-(1H-imidazol-1-yl)propyl]amine / 1-methoxy-2-amino-4-(2-hydroxyethylamino)benzene;
N-(4-amino-3-methylphenyl)-N-[3-(1H-imidazol-1-yl)propyl]amine / 2-amino-3-hydroxypyridine;
N-(4-amino-3-methylphenyl)-N-[3-(1H-imidazol-1-yl)propyl]amine / 1-naphthol;
4,5-diamino-1-(2-hydroxyethyl)pyrazole / resorcinol;
4,5-diamino-1-(2-hydroxyethyl)pyrazole / 2-methylresorcinol;
4,5-diamino-1-(2-hydroxyethyl)pyrazole / 5-amino-2-methylphenol;
4,5-diamino-1-(2-hydroxyethyl)pyrazole / 3-aminophenol;
4,5-diamino-1-(2-hydroxyethyl)pyrazole / 2-(2,4-diaminophenoxy)ethanol;
4,5-diamino-1-(2-hydroxyethyl)pyrazole / 1,3-bis(2,4-diaminophenoxy)propane;
4,5-diamino-1-(2-hydroxyethyl)pyrazole / 1-methoxy-2-amino-4-(2-hydroxyethylamino)benzene;
4,5-diamino-1-(2-hydroxyethyl)pyrazole / 2-amino-3-hydroxypyridine;
4,5-diamino-1-(2-hydroxyethyl)pyrazole / 1-naphthol.

## Revendications

1. Procédé de coloration par oxydation de fibres de kératine, en particulier de cheveux, **caractérisé en ce que**
a) un agent de prétraitement qui contient au moins une silicone 4-morpholinométhyle-substituée de la formule (V), 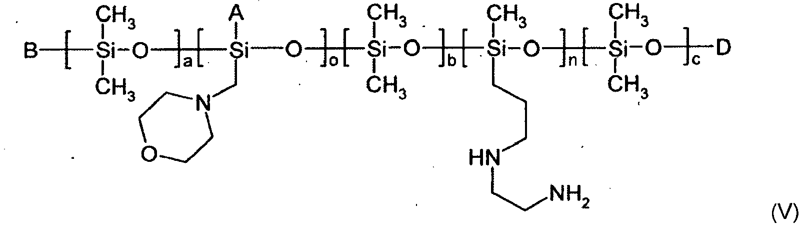 dans laquelle
A représente une unité structurelle (I), (II), (IIIa), (IIIb), (IIIe) ou (IIIf) liée par un -O-, 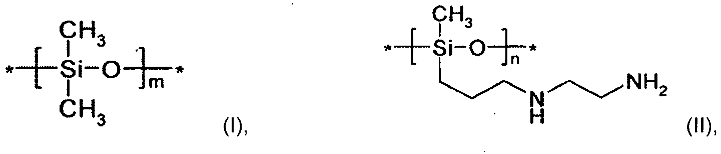 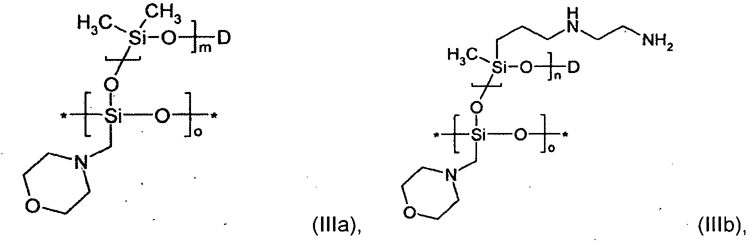 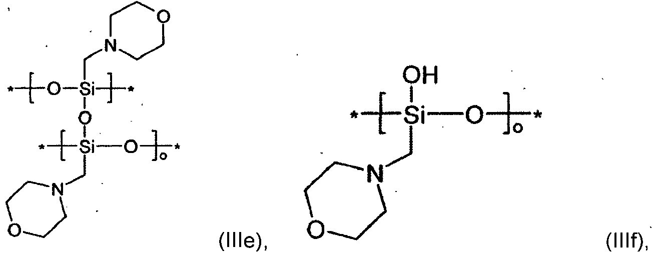 ou représente un radical oligomère ou polymère, lié par un -O-, contenant des unités structurelles de formules (I), (II), (IIIa), (IIIb), (IIIe) et (IIIf), ou représente -OH,
* représente une liaison à l'une des unités structurelles (I), (II), (IIIa), (IIIb), (IIIe) et (IIIf), ou représente un groupe d'extrémité B (lié par Si) ou D (lié par O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
a, b et c sont des nombres entiers de 0 à 990, avec la condition a + b + c > 0
m, n et o sont des nombres entiers de 1 à 990,
est appliqué sur les fibres de kératine, notamment les cheveux,
b) ensuite, pendant une durée d'une seconde à 24 heures après l'étape a) un agent de coloration capillaire est appliqué sur les fibres de kératine,
l'agent de coloration capillaire étant obtenu par mélange d'une composition (A), qui contient au moins un précurseur de colorant par oxydation du type développeur et au moins un précurseur de colorant par oxydation du type copulateur, avec une composition (B) qui contient au moins un agent d'oxydation,
**caractérisé en ce que** la composition (B) contient au moins une huile cosmétique dans une quantité totale de 10 à 80% en poids, par rapport au poids de la composition (B).

2. Procédé selon la revendication 1, **caractérisé en ce que** l'agent de prétraitement, utilisé à l'étape a, contient au moins une silicone 4-morpholinométhyl-substituée de la formule (V), dans laquelle on a
m > (n + o) ou (a + b + c) > (n + o), de préférence
m > 8 (n + o) ou (a + b + c) > 8 (n + o), plus préférablement
m > 25 (n + o) ou (a + b + c) > 25 (n + o), plus préférablement
m > 77 (n + o) ou (a + b + c) > 77 (n + o), et notamment
m > 200 (n + o) ou (a + b + c) > 200 ( n + o).

3. Procédé selon l'une des revendications 1 ou 2, **caractérisé en ce que** l'agent de prétraitement utilisé à l'étape a contient, sur la base de son poids, une ou plusieurs silicones 4-morpholinométhyl-substituées dans une quantité totale de 0,001 à 5% en poids, de préférence de 0,005 à 2% en poids, de façon particulièrement préférée de 0,01 à 1% en poids, de façon extraordinairement préférée de 0,02 à 0,1%, à chaque fois sur la base du poids total de l'agent de prétraitement.

4. Procédé selon l'une des revendications 1 à 3, **caractérisé en ce que** l'agent de prétraitement utilisé à l'étape a contient une ou plusieurs silicones 4-morpholinométhyl-substituées à terminaison hydroxyle, dans lesquelles le rapport molaire hydroxyle/alcoxyle est dans la gamme de 0,2:1 à 0,4:1, de préférence dans la gamme de 1:0,8 à 1:1,1.

5. Procédé selon l'une des revendications 1 à 4, **caractérisé en ce que** la masse molaire moyenne en poids de la silicone 4-morpholinométhyl-substituée utilisée à l'étape a de la formule (V) est dans la gamme de 2000 à 1000000 gmol⁻¹, de préférence dans la gamme de 5000 à 200000 gmol⁻¹.

6. Procédé selon l'une des revendications 1 à 5, **caractérisé en ce que** la silicone 4-morpholinométhyl-substituée utilisée à l'étape a de la formule (V) se présente sous la forme d'une émulsion huile-dans-eau dans laquelle la taille moyenne en nombre des particules de silicone dans l'émulsion est de préférence dans la gamme de 3 à 500 nm, de manière particulièrement préférée dans la gamme de 5 à 60 nm.

7. Procédé selon l'une des revendications 1 à 6, **caractérisé en ce que** l'au moins une huile cosmétique, qui est contenue dans la composition (B) dans une quantité totale de 10 à 80% en poids par rapport au poids de la composition (B), est choisie parmi les hydrocarbures naturels et synthétiques, de façon particulièrement préférée les huiles de paraffine, les isoparaffines en C₁₈ à C₃₀, en particulier l'isoeicosane, les polyisobutènes et les polydécènes, les isoparaffines en C₈ à C₁₆, et le 1,3-di-(2-éthylhexyl)-cyclohexane ; les esters d'alcanols en C₈ à C₂₂ linéaire ou ramifié avec de l'acide benzoïque ; les alcools gras ayant 6 à 30 atomes de carbone qui sont insaturés ou ramifiés et saturés ou ramifiés et insaturés ; les triglycérides d'acides gras en C₈ à C₃₀ linéaires ou ramifiés, saturés ou insaturés éventuellement hydroxylés, en particulier les huiles naturelles ; les esters d'alcanols C₂ à C₁₀ linéaires ou ramifiés avec l'acide dicarboxylique ; les esters des alcools gras linéaires ou ramifiés saturés ou insaturés ayant 2 à 30 atomes de carbone avec des acides gras linéaires ou ramifiés saturés ou insaturé ayant 2 à 30 atomes de carbone qui peuvent être hydroxylés ; les produits d'addition de 1 à 5 unités de propylène à des alcanols en C₈ à C₂₂ monovalent ou polyvalent ; les produits d'addition d'au moins 6 unités oxyde d'éthylène et/ou oxyde de propylène à des alcanols en C₃ à C₂₂ monovalents ou polyvalents ; les esters d'acides hydroxycarboxyliques en C₂ à C₇ monovalent ou polyvalent avec des alcools gras en C₈ à C₂₂ ; les esters symétriques, asymétriques ou cycliques de l'acide carbonique avec des alcanols en C₃ à C₂₂, des alcanediols en C₃ à C₂₂ ou des alcanetriols en C₃ à C₂₂ ; les esters de dimères d'acides gras en C₁₂ à C₂₂ insaturés (acides gras dimères) avec des alcanols en C₂ à C₁₈ linéaires, ramifiés ou cycliques monovalents ou avec des alcanols en C₂ à C₆ linéaires ou ramifiés polyvalents ; les huiles de silicone et des mélanges des substances susmentionnées.

8. Procédé selon l'une des revendications 1 à 7, **caractérisé en ce que** la composition (B) contient au moins une huile cosmétique dans une quantité totale de 12 à 70% en poids, de préférence de 14 à 60% en poids, de manière particulièrement préférée de 15 à 52% en poids et de manière extraordinairement préférée de 17 à 35% en poids, à chaque par rapport au poids de la composition (B).

9. Procédé selon l'une revendications 1 à 8, **caractérisé en ce que** la composition (B) contient :
de 1 à 24% en poids, de préférence de 4 à 10% en poids, de manière particulièrement préférée de 3 à 6% en poids, de peroxyde d'hydrogène (calculé sous la forme de H₂O₂ à 100%), en outre
au moins une huile cosmétique dans une quantité totale de 12 à 70% en poids, de préférence de 14 à 60% en poids, de manière particulièrement préférée de 15 à 52% en poids, et de manière extraordinairement préférée de 17 à 35% en poids, en outre
au moins un tensioactif dans une quantité totale de 0,1 à 5% en poids, de préférence de 0,5 à 3% en poids et de manière particulièrement préférée de 1 à 2% en poids, et
au moins un alcanol saturé linéaire ayant 12 à 30 atomes de carbone dans une quantité totale de 0,1 à 10% en poids, de préférence de 0,5 à 7% en poids et de manière particulièrement préférée de 3 à 5% en poids , toutes les données en % en poids se rapportant au poids de la composition (B).

10. Procédé selon l'une des revendications 1 à 9, **caractérisé en ce que** l'agent de coloration capillaire prêt à l'emploi utilisé à l'étape de procédé b) contient au moins une huile cosmétique dans une quantité totale de 5 à 50% en poids, de préférence de 8 à 40% en poids, de manière particulièrement préférée de 12 à 30% en poids, de manière extraordinairement préférée de 15 à 25% en poids, à chaque fois par rapport au poids de l'agent de coloration capillaire.

11. Procédé selon l'une des revendications 1 à 10, **caractérisé en ce qu'**on laisse agir l'agent de pré traitement, appliqué à L'étape de procédé a, pendant une durée de 2 secondes à 120 minutes, de préférence de 5 secondes à 10 minutes, sur les cheveux avant d'effectuer au moins une des étapes de procédé suivantes a)i) qui sont effectuées avant l'étape de procédé b) :
rincer les cheveux ;
sécher les cheveux avec une serviette,
laisser sécher les cheveux à l'air,
sécher les cheveux au sèche-cheveux,
sécher les cheveux au casque sèche-cheveux,
des combinaisons des étapes de procédé susmentionnées,
le procédé de séchage étant de préférence effectué à une température de 20°C à 150°C

12. Procédé selon la revendication 11, **caractérisé en ce qu'**aucun rinçage des cheveux n'est effectué entre l'application de l'agent de prétraitement, effectuée à l'étape de procédé a, et le processus de séchage ou les processus de séchage.

13. Procédé selon l'une des revendications 1 à 12, **caractérisé en ce que** la durée entre les étapes de procédé a et b est de 30 secondes à 20 minutes, de préférence de 45 secondes à 10 minutes, de manière particulièrement préférée de 1 à 5 minutes.

14. Procédé selon l'une des revendications 1 à 13, **caractérisé en ce qu'**une combinaison, appliquée à l'étape b, d'au moins un précurseur de colorant par oxydation du type développeur et d'au moins un précurseur de colorant d'oxydation du type copulateur est choisie parmi au moins une des combinaisons suivantes, les composés aminés et les composés hétérocycliques azotés pouvant se présenter également sous la forme de leurs sels physiologiquement acceptables :
p-toluènediamine/résorcinol ;
p-toluènediamine/2-méthylrésorcinol ;
p-toluènediamine/5-amino-2-méthylphénol ;
p-toluènediamine/3-aminophénol ;
p-toluènediamine/2-(2,4-diaminophénoxy)éthanol ;
p-toluènediamine/1,3-bis(2,4-diaminophénoxy)propane ;
p-toluènediamine/1-méthoxy-2-amino-4-(2-hydroxyéthylamino)benzène ;
p-toluènediamine/2-amino-3-hydroxypyridine ;
p-toluènediamine/1-naphtol ;
2-(2-hydroxyéthyl)-p-phénylènediamine/résorcinol ;
2-(2-hydroxyéthyl)-p-phénylènediamine/2-méthylrésorcinol ;
2-(2-hydroxyéthyl)-p-phénylènediamine/5-amino-2-méthylphénol ;
2-(2-hydroxyéthyl)-p-phénylènediamine/3-aminophénol ;
2-(2-hydroxyéthyl)-p-phénylènediamine/2-(2,4-diaminophénoxy)éthanol ;
2-(2-hydroxyéthyl)-p-phénylènediamine/1,3-bis(2,4-diaminophénoxy)propane ;
2-(2-hydroxyéthyl)-p-phénylènediamine/1-méthoxy-2-amino-4-(2-hydroxyéthylamino)benzène ;
2-(2-hydroxyéthyl)-p-phénylènediamine/2-amino-3-hydroxypyridine ;
2-(2-hydroxyéthyl)-p-phénylènediamine/1-naphtol ;
2-méthoxyméthyl-p-phénylènediamine/résorcinol ;
2-méthoxyméthyl-p-phénylènediamine/2-méthylrésorcinol ;
2-méthoxyméthyl-p-phénylènediamine/5-amino-2-méthylphénol ;
2-méthoxyméthyl-p-phénylènediamine/3-aminophénol ;
2-méthoxyméthyl-p-phénylènediamine/2-(2,4-diaminophénoxy)éthanol ;
2-méthoxyméthyl-p-phénylènediamine/1,3-bis(2,4- diaminophénoxy)propane ;
2-méthoxyméthyl-p-phénylènediamine/1-méthoxy-2-amino-4-(2-hydroxyéthylamino)benzène ;
2-méthoxyméthyl-p-phénylènediamine/2-amino-3-hydroxypyridine ;
2-méthoxyméthyl-p-phénylènediamine/1-naphtol ;
N-(4-amino-3-méthylphényl)-N-[3-(1H-imidazol-1-yl)propyl]amine/résorcinol ;
N-(4-amino-3-méthylphényl)-N-[3-(1H-imidazol-1-yl)propyl]amine/2-méthylrésorcinol ;
N-(4-amino-3-méthylphenyl)-N-[3-(1H-imidazol-1-y)propyl]amine/5-amino-2-méthylphénol ;
N-(4-amino-3-méthylphényl)-N-[3-(1H-imidazol-1-yl)propyl]amine/3-aminophénol ;
N-(4-amino-3-méthylphényl)-N-[3-(1H-imidazol-1-yl)propyl]amine/(2-(2,4-diamino-phénoxy)-éthanol ;
N-(4-amino-3-méthylphényl)-N-[3-(1H-imidazol-1-yl)propyl]amine/1,3-bis(2,4-diaminophénoxy)propane ;
N-(4-amino-3-méthylphényl)-N-[3-(1H-imidazol-1-yl)propyl]amine/1-méthoxy-2-amino-4-(2-hydroxyéthylamino)benzène ;
N-(4-amino-3-méthylphényl)-N-[3-(1H-imidazol-1-yl)propyl]amine/2-amino-3-hydroxypyridine ;
N-(4-amino-3-méthylphényl)-N-[3-(1H-imidazol-1-yl)propyl]amine/1-naphtol ;
4,5-diamino-1-(2-hydroxyéthyl)pyrazole/résorcinol ;
4,5-diamino-1-(2-hydroxyéthyl)pyrazole/2-méthylrésorcinol ;
4,5-diamino-1-(2-hydroxyéthyl)pyrazole/phénol/5-amino-2-méthylphénol ;
4,5-diamino-1-(2-hydroxyéthyl)pyrazole/3-aminophénol ;
4,5-diamino-1-(2-hydroxyéthyl)pyrazole/2-(2,4-diaminophénoxy)éthanol ;
4,5-diamino-1-(2-hydroxyéthyl)pyrazole/1,3-bis(2,4-diaminophénoxy)propane ;
4,5-diamino-1-(2-hydroxyéthyl)pyrazole/1-méthoxy-2-amino-4-(2-hydroxyéthylamino)benzène ;
4,5-diamino-1-(2-hydroxyéthyl)pyrazole/2-amino-3-hydroxypyridine ;
4,5-diamino-1-(2-hydroxyéthyl)pyrazole/1-naphtol.
